# EUROPEAN PATENT SPECIFICATION

(11) **EP 2 201 107 B1**
(45) Date of publication and mention of the grant of the patent: **16.01.2019**
(21) Application number: 08876854.4
(22) Date of filing: 28.07.2008
(51) Int. Cl.: C07K 14/00, G01N 33/48, C12N 9/78, A61K 38/00

(54) **NOVEL GTP CYCLOHYDROLASE TYPE IB**
NEUE GTP-CYCLOHYDROLASE VOM TYP IB
NOUVELLE GTP CYCLOHYDROLASE DE TYPE IB

(30) Priority: 26.07.2007 US 935124 P
(43) Date of publication of application: 30.06.2010
(73) Proprietor: Western University Of Health Sciences, Pomona, CA 91766 (US)
(72) Inventor: SWAIRJO, Manal A., Pomona, CA 91766 (US); IWATA-REUYL, Dirk, Portland, OR 97201 (US); DE CRECY-LAGARD, Valerie, Gainesville, FL 32611 (US)
(74) Representative: Jacobi, Markus Alexander
(86) International application number: PCT/US2008/071358
(87) International publication number: WO 2010/039115

(56) References cited:
- WO-A2-02/077183
- US-A1- 2005 136 404
- B. EL YACOUBI ET AL: "Discovery of a New Prokaryotic Type I GTP Cyclohydrolase Family", JOURNAL OF BIOLOGICAL CHEMISTRY, vol. 281, no. 49, 8 December 2006 (2006-12-08), pages 37586-37593, XP055220884, US ISSN: 0021-9258, DOI: 10.1074/jbc.M607114200
- B. SANKARAN ET AL: "Zinc-Independent Folate Biosynthesis: Genetic, Biochemical, and Structural Investigations Reveal New Metal Dependence for GTP Cyclohydrolase IB", JOURNAL OF BACTERIOLOGY, vol. 191, no. 22, 15 November 2009 (2009-11-15), pages 6936-6949, XP055270631, US ISSN: 0021-9193, DOI: 10.1128/JB.00287-09
- EI YACOUBI ET AL.: 'Discovery of a New Prokaryotic Type I GTP Cyclohydrolase Family.' J BIOL CHEM. vol. 281, no. 49, 2006, pages 37586 - 37593, XP055220884

## Description

This invention was made with United States government support under Grant R01 GM70641-01 awarded by the National Institutes of Health, and Award No. MCB-0516948 awarded by the National Science Foundation. The Stanford Synchrotron Research Laboratory Structural Molecular Biology Program is supported by the Department of Energy, National Institutes of Health, and the National Institute of General Medical Sciences. The government has certain rights in the invention.

### RELATED APPLICATIONS

The present application is related to and claims the priority benefit of U.S. Provisional Patent Application Ser. No. 60/935,124, entitled "Novel GTP Cyclohydrolase Type IB," filed July 26, 2007.

### BACKGROUND

Bacterial diseases, such as those caused by *Neisseria gonorrhea* and *Staphylococcus aureus*, pose significant disease and health risks. *N. gonorrhoeae* is the causative agent of the sexually transmitted disease gonorrhea (infection of the genitalia, and urinary tract) and cause of pelvic inflammatory disease and infertility in one million women a year. Worldwide, there are an estimated 62 million new cases a year, with an average of 22 million cases at any given time. About 0.8 million new gonorrhea infections are reported each year in the U.S, primarily among teenage females and African Americans, costing $1.1 billion/year in treatment and related expenses. Importantly, gonorrhea infections increase the transmission and susceptibility to human immunodeficiency virus infection. Moreover, gonorrhea has become resistant to traditional treatments with quinolone (ciprofloxacin), tetracycline, penicillin, and sulfonamides. Currently, twenty percent of reported gonorrhea cases in the U.S. and Europe are resistant to all of these drugs, with the highest resistance seen among homosexual males. Although gonorrhea can now be treated with ultra high doses of azithromycin, resistance to that antibiotic is also emerging.

The *S. aureus* bacterium currently causes the most common and serious infections that occur in hospitalized patients. In recent years, *S. aureus* has become resistant to antibiotics (thus named Multidrug- or Methicillin-Resistant *S. aureus*, MRSA), causing a serious public health problem in the United States and worldwide. Sixty percent of intensive-care-unit infections in the U.S. are caused by MRSA, leading to significant mortality. The MRSA "superbug" multiplies very rapidly in the bloodstream causing toxic shock syndrome, and/or on the skin causing furuncles. Once an infection occurs, it is almost impossible to treat with existing antibiotics, especially in immune-compromised and elderly patients. When an incurable MRSA infection reaches the heart, it often causes fatal endocarditis.

WO 2002/077183 describes the identification of essential genes in microorganisms including sequences from *Neisseria gonorrhoeae.*

Hence, there is a growing need for alternative treatments against such bacterial pathogens.

### SUMMARY OF THE INVENTION

An object of the present invention provides for a novel avenue for combating bacterial pathogens by targeting specific enzymes that archaea and bacteria, but not eukarya, require. The present application presents a novel, purified bacterial enzyme, GTP Cyclohydrolase Type IB (GCYH-IB).

The disclosure further provides for the crystalline structure of GCYH-IB. The present invention relates to a GCYH-IB crystal according to claim 1. The application further discloses a computer-readable medium having GTP Cyclohydrolase Type IB crystal structure information stored thereon.

### DESCRIPTION OF THE DRAWINGS

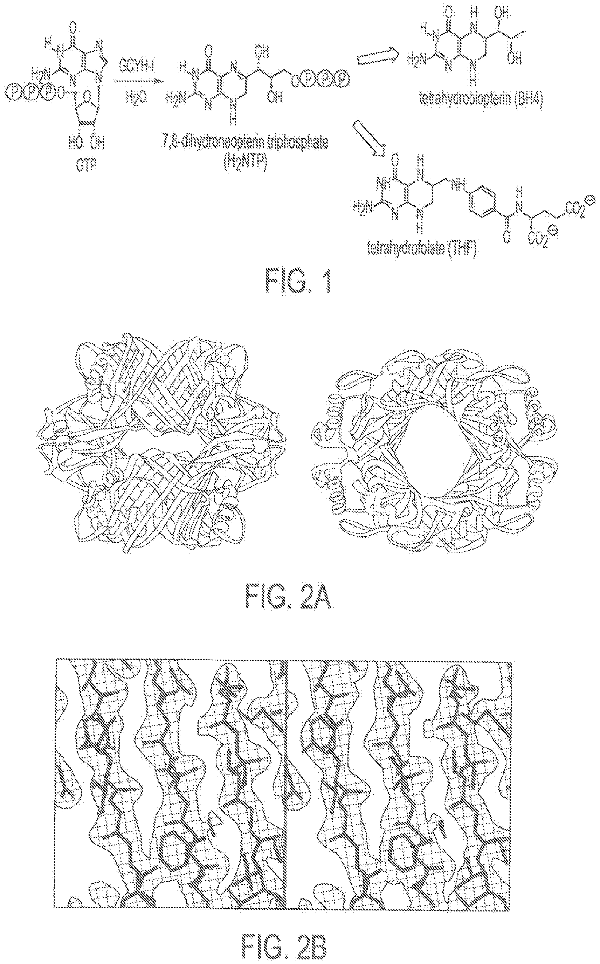

Figure 1 presents a diagram of the reaction catalyzed by GTP Cyclohydrolase Type I (GCYH-I), and the metabolic fate of H₂NTP.
Figure 2 shows the overall structure of GTP Cyclohydrolase Type IB (GCYH-IB). Panel 2A is a ribbon diagram showing a side view (left) and a top view (right) of the full biological tetramer. Panel 2B is a stereoview of a FOM-weighted experimental electron density map (resolution 2.2 Å, contour level 1.5 σ), calculated after solvent flattening, in the β-sheet region. The map is superimposed on the refined model. The figure was prepared with *BobScript* software. Esnouf, 55(4) Acta Crystallogr. D Biol. Crystallogr. 938-40 (1999).

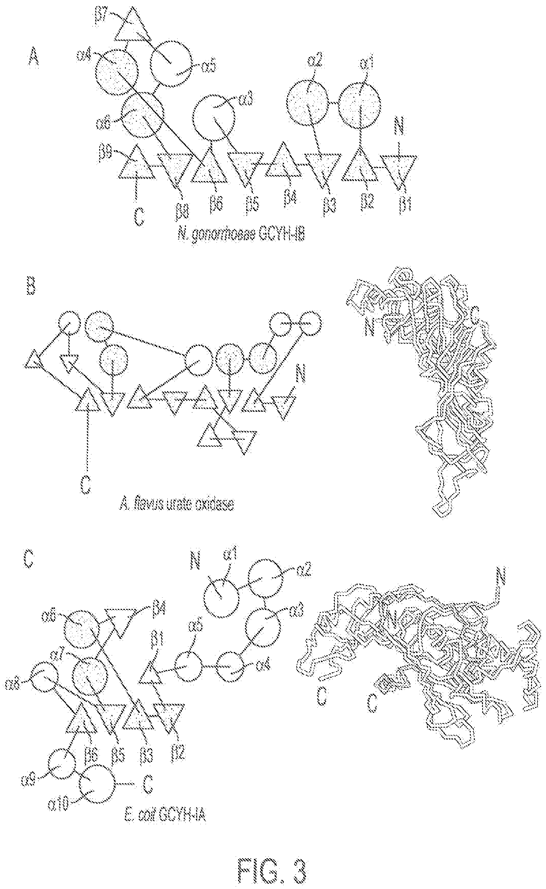

Figure 3 depicts the topology of the GCYH-IB monomer and structural comparison with other T-fold enzymes. Panel 3A presents topology diagrams of *N. gonorrhoeae* GCYH-IB. Panel 3B: of *A. flavus* urate oxidase; Panel 3C: *of E. coli* GCYH-IA. Michalopoulos et al., 32 Nucleic Acids Res. D251-54 (2004). The N- and C-terminal modules of the core bimodular T-fold are colored in gray and light gray, respectively. C_{α} trace superpositions of the N- (gray) and C-terminal (light gray) modules of GCYH-IB with corresponding enzymes are shown in Panel B and panel C. Secondary structure nomenclature is shown for the two GCYH-I enzymes.

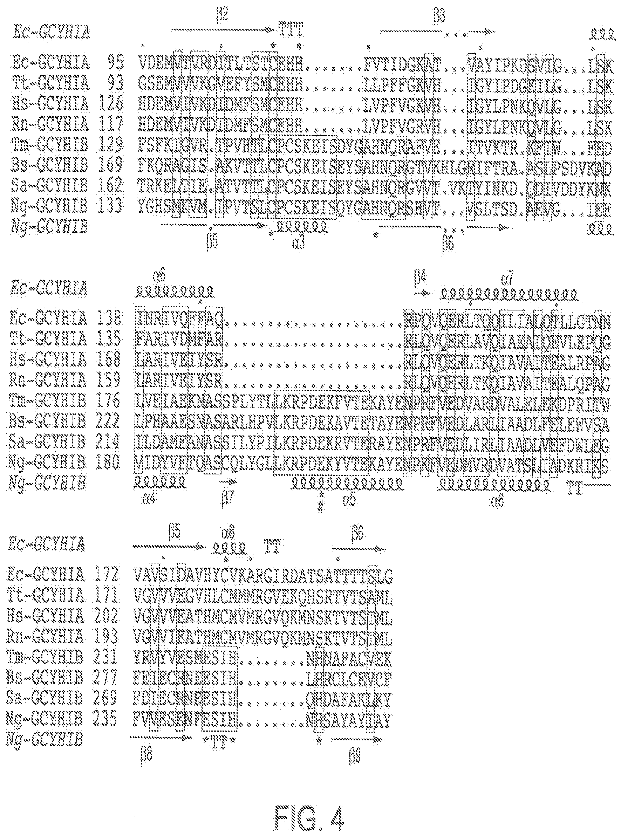

Figure 4 presents structure-guided multi-sequence alignment of GCYH-IA and GCYH-IB in the shared T-fold region. For clarity, only four sequences are shown from each subfamily. Residue labels and secondary structure elements (from the crystal structures) are shown above and below the sequence alignment for enzyme subfamilies A and B, respectively. Secondary structure nomenclature is as in Fig. 3. Residues conserved between the two subfamilies are vertically outlined, with the strictly conserved residues (the substrate binding Glu and zinc binding Cys) highlighted in dark gray. Conserved regions within GCYH-IB subfamily are boxed. The zinc ion-liganding side chains in each subfamily are labeled with gray stars. The Mn-liganding side chains in GCYH-IB are labeled with black stars. # indicates a zinc liganding side chain from the neighboring subunit. Ec: *Escherichia coli,* Tt: *Thermus thermophilus*, Hs: *Homo sapien*, Rn: *Rattus norvegicus,* Tm: *Thermotoga maritima*, Bs: *Bacillus subtilis,* Sa: *Staphylococcus aureus*, Ng: *Neisseria gonorrhoeae.*

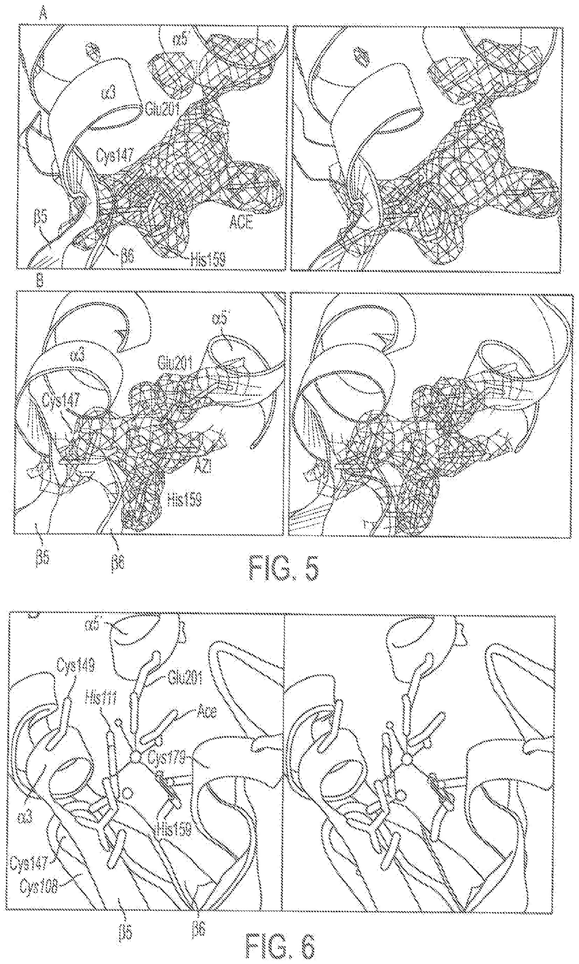

Figure 5 depicts the metal site in GCYH-IB. Panel A shows a stereoview of annealed Fo-Fc electron density map (1500 K, 2.2 Å, 2.5 σ, Zn²⁺ and its ligands omitted from the phase calculation) superimposed on the model. Panel B shows the Mn²⁺-occupied metal site in the GCYH-IB•Mn²⁺ complex. Stereoview of annealed omit Fo-Fc electron density map (1500 K, 2.04 Å, 2.5 σ, Mn²⁺ and its ligands omitted from phase calculation) superposed on the model. Metal ions and water molecules are shown as spheres. Secondary structure elements in GCYH-IB are labeled.
Figure 6 presents a 3D alignment of GCYH-IB (regular labels) and *T. thermophilus* GCYH-IA (italic labels) in the active-site region. Strictly conserved Cys149 in the metal binding loop of GCYH-IB is shown although it does not interact with the bound metal.

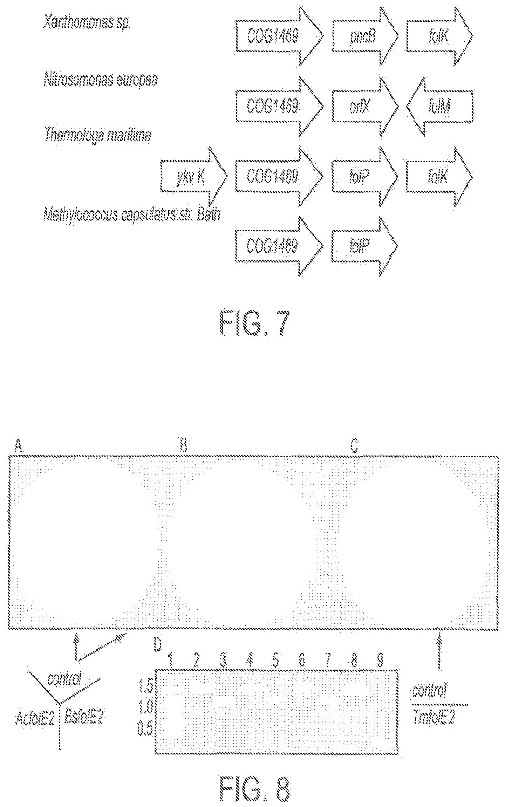

Figure 7 illustrates the physical clustering of GCYH-IB (labeled COG1469) encoding genes with folate biosynthetic genes, such as folK, folP, and folM (an alternative dihydrofolate reductase gene). Rebelo et al., 326(2) J. Mol. Biol. 503-16 (2003).
Figures 8 depicts various complementation data of the dT auxotrophy on LB (Panel A) or the slow growth phenotype on LB dT (Panel B) of the *E. coli* folE::KanR by pBAD derivatives expressing the GCYH-IB genes from *B. subtilis* (BsfolE2) and *A. baylyi* (AcfolE2). Panel C shows complementation of the slow growth on LB in the presence of dT of the *E. coli* folE::KanR by a plasmid expressing TM0039 (TmfolE2). The control is folE::KanR transformed by pBAD24. The plates were incubated at 37°C for 48 h. Panel D presents PCR amplification to check for the presence of the folE::KanR allele (lanes 2, 4, 6, and 8) and to check for the presence of an insert of the expected size in the pBAD derivatives (lanes 3, 5, 7, and 9) was performed on the folE::KanR strain transformed with plasmids expressing AcfolE2 (lanes 2 and 3), BsfolE2 (lanes 4 and 5), and TmfolE2 (lanes 6 and 7) and with the pBAD24 control (lanes 8 and 9). The expected size of the PCR product detecting folE::KanR is about3.5 kb, whereas the same primers amplify a 2.5-kb product in the wild type strain. The sizes of the PCR products resulting from having AcfolE2, BsfolE2,or TmfolE2 in pBAD24 are 1072, 1105, and 960 bp, respectively.

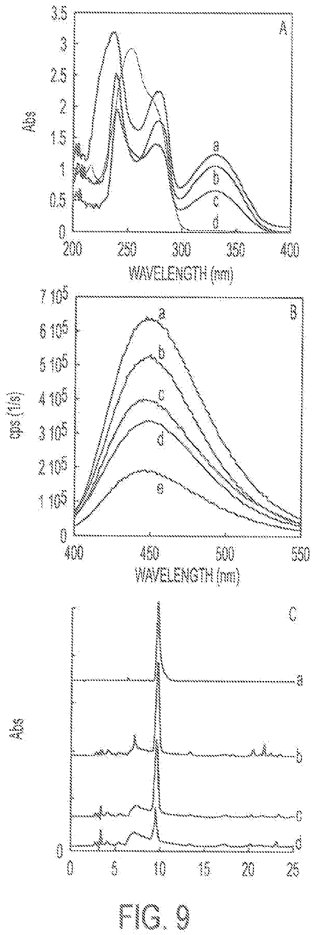

Figure 9 graphically depicts GCYH-I activity assays. Panel 9A shows UV-visible spectra of cyclohydrolase assays. Line a, *E. coli* FolE; b, GCYH-IB protein from *N. gonorrhoeae;* c, GCYH-IB protein *from B. subtilis;* d, no enzyme. Panel 9B reflects fluorescence spectra of authentic neopterin and cyclohydrolase assays following post-reaction dephosphorylation and oxidation. Fluorescence was measured with excitation at 365 nm. Line a, *T. maritima* GCYH-IB; b, *B. subtilis* GCYH-IB; line c, *E. coli* FolE; d, authentic neopterin; e, *N. gonorrhoeae* GCYH-IB. Panel 9C presents HPLC chromatograms of authentic neopterin (line a) and cyclohydrolase assays with *E. coli* FolE (line b), *B. subtilis* GCYH-IB added to authentic neopterin (line c), and *B. subtilis* GCYH-IB alone (line d). Enzyme assays were subjected to postreaction dephosphorylation and oxidation.

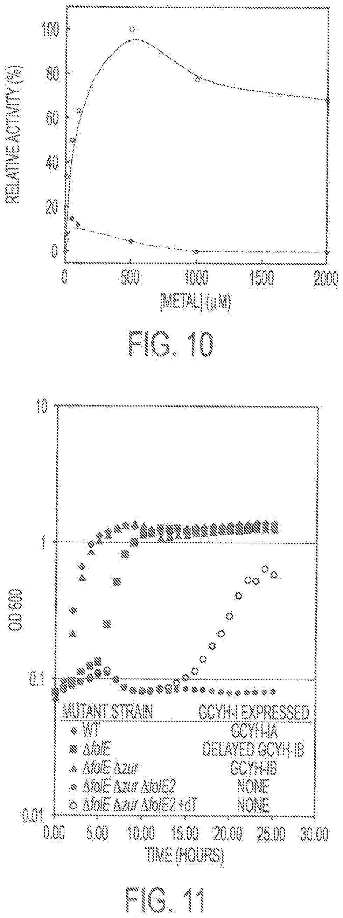

Figure 10 presents *B. subtilis* GCYH-IB enzymatic activity versus [Mn²⁺] (○) or [Zn²⁺] (●). Each data point represents the average of four sets of triplicate assays.
Figure 11 reflects GCYH-IB-dependent complementation of the *B. subtilis* Δ*folE* mutant strain. Growth curves in rich medium of wild type (WT) and mutant *B. subtilis* strains expressing either type of GCYH-I. Strains and their corresponding expressed GCYH-I isozyme are listed.

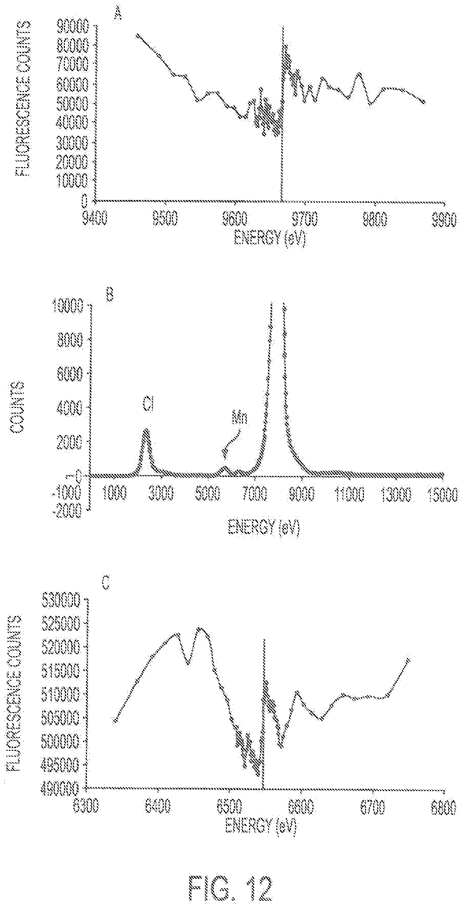

Figure 12 shows data from X-ray emission and fluorescence scans of bound metals in GCYH-IB crystals. Panel A reflects the fluorescence spectrum from crystal GCYH-IB taken near the zinc absorption edge. Panels B and C are emission scan and fluorescence spectrum, respectively, from GCYH-IB•Mn²⁺ crystal taken near the manganese K-shell absorption edge. Inflection points in the fluorescence spectra are indicated with vertical lines. The large peak in panel B corresponds to scattering from the main beam.

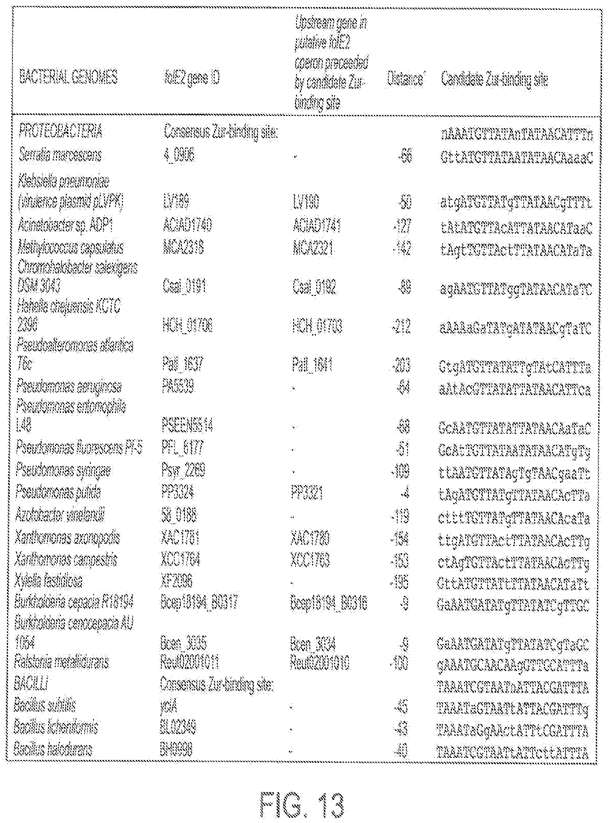

Figure 13 is a Table of candidate Zur-binding sites upstream of *folE2* in bacterial genomes. ^{a}Distance is given in nucleotides relative to the start codon of the downstream gene.

### DETAILED DESCRIPTION

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

As used herein and in the claims, the singular forms "a," "an," and "the" include the plural reference and equivalents known to those skilled in the art unless the context clearly indicates otherwise. Other than in the operating examples, or where otherwise indicated, all numbers expressing quantities of ingredients or reaction conditions used herein should be understood as modified in all instances by the term "about."

All patents and other publications identified are for the purpose of describing and disclosing, for example, the methodologies described in such publications that might be used in connection with the present invention, but are not to provide definitions of terms inconsistent with those presented herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on information available to the applicants and do not constitute any admission as to the correctness of the dates or contents of these documents.

Unless defined otherwise, all technical and scientific terms used herein have the same meaning as those commonly understood to one of ordinary skill in the art to which this invention pertains. When COG1469 is used in the context of a protein or gene family, it is synonymous with GCYH-IB protein or gene family.

Folic acid or folate is an essential metabolite in all life forms. It is used by all cells for the biosynthesis of purines (building blocks of DNA and RNA), amino acids, and vitamins. Unlike humans, who need folic acid as a dietary supplement, bacteria and yeast biosynthesize folic acid *de novo.* As a result, folate biosynthesis in bacteria and yeast has become an attractive target in antibacterial therapeutics. For example, sulfonamides, the first synthetic drugs developed with broad antibacterial activity, target a late step in the bacterial folate biosynthetic pathways. Emerging resistance to existing antibiotics spurs the quest for new and more effective target points along the pathway. One such point is the first step in folate biosynthesis: the conversion of GTP to 7,8-dihydroneopterin triphosphate by the enzyme GTP cyclohydrolase I (GCYH-I). The presence in humans of a homologous enzyme (required for making biopterin, another metabolic co-factor distinct from folate), complicated the development of bacterial GCYH-I as a drug target.

The present application provides for a novel GCYH-I family of enzymes, called GCYH-IB, that has been identified in pathogenic bacteria. El Yacoubi et al., 281(49) J. Biol. Chem. 37586-93 (2006). Importantly, GCYH-IB is absent in humans. The present invention identifies the biochemical properties and crystal structure of GCYH-IB from the pathogenic bacterium *N. gonorrhoeae.* The unique structure of the active center and the new metal dependency of this cyclohydrolase suggest an enzymatic mechanism distinct from that of the canonical GCYH-I. The differences in the active-site architecture and enzymatic mechanism of the two subtypes of GCYH-I enzymes can be exploited for the design of selective inhibitors of the bacteria-specific enzyme. Realization of such a goal allows for development of GCYH-IB as a therapeutic target for antibacterial pharmaceuticals. Furthermore, the presence of GCYH-IB as the sole GTP-cyclohydrolase in two clinically important human pathogens, *N. gonorrhoeae* and *S. aureus*, allows investigations of this enzyme as a potential drug target against the antibiotic resistant forms of gonorrhea and MRSA infections.

More specifically, regarding folate biosynthesis, folic acid in the form of various tetrahydrofolate (THF) derivatives serves as a cofactor in one-carbon transfer reactions during the synthesis of purines, thymidylate, pantothenate, glycine, serine and methionine, in all kingdoms of life. Nichol et al., 54 Ann. Rev. Biochem. 729-64 (1985). In bacteria, THF is also involved in the biosynthesis of the initiator formylmethionyl-tRNA. Clark & Marker, 17(2) J. Mol. Biol. 394-406 (1966). Plants, fungi, and most bacteria synthesize THF *de novo* from GTP and *p*-aminobenzoic acid (pABA). Green et al., in E. COLI & SALMONELLA, CELLULAR & MOLECULAR BIOL. 665-73 (Neidhart, ed., Am. Soc. Micro., Washington, DC, 1996); Cossins & Chen, 45(3) Phytochem. 437-52 (1997); Hanson & Gregory, 5(3) Curr. Opin. Plant Biol. 244-49 (2002). Animals lack key enzymes of the folate biosynthetic pathway, and thus require a dietary source of folate for normal growth and development. Lucock, 71(1) Mol. Genet. Metab. 121-38 (2000).

GTP cyclohydrolase I (GCYH-1; EC 3.5.4.16) is the first enzyme of the *de novo* THF pathway (Fig. 1). Nichol et al., 1985. It is encoded in *E. coli* by the *folE* gene (Katzenmeier et al., 372(11) Biol. Chem. Hoppe Seyler 991-97 (1991); Schoedon et al., 210(2) Eur. J. Biochem. 561-68 (1992)), and catalyzes a complex reaction that begins with hydrolytic ring opening of the purine ring at C₈ to generate an N-formyl intermediate, which is then the site for a second hydrolysis with concomitant loss of C₈ as formic acid. Yim & Brown 251(16) J. Biol. Chem. 5087-94 (1976). In the subsequent steps of the reaction, the ribosyl moiety undergoes ring-opening and an Amidori rearrangement, followed by cyclization to generate the pterin ring in THF (Fig.1). The product of GCYH-1, 7,8-dihydroneopterin triphosphate (H₂NTP), is subsequently dephosphorylated to 7,8-dihydroneopterin by both specific and non specific phosphatases (Klaus et al., 280(7) J. Biol. Chem. 5274-80 (2005)), and the remainder of THF biosynthesis carried out by the enzymes encoded by the *folBKPCA* genes (in *E. coli*)*.* Green et al., 1996.

The folate pathway has a storied history as an important target in antibacterial therapeutics and cancer chemotherapy. Dihydropteroate synthase is the target of the sulfonamides (Huovinen et al., 39(2) Antimicrobial Agents & Chemotherapeutics, 279-89 (1995)), and dihydrofolate reductase is the target of methotrexate, the first anticancer chemotherapy developed. A homologous GCYH-I is found in mammals and other higher eukaryotes, however, where it catalyzes the first step of the biopterin (BH4) pathway (Fig. 1), an essential cofactor in aromatic amino acid oxidation in the biosynthesis of tyrosine and neurotransmitters such as serotonin and L-DOPA. Thony et al., 347(1) Biochem. J. 1-16 (2000); Bonafe et al., 69(2) Am. J. Human Genet. 269-77 (2001). Although several enzymes in the folate pathway have proved important antimicrobial targets (Huovinen et al., 1995), the presence of homologous GCYH-I enzymes in both humans and bacteria has precluded the development of GCYH-I as a viable target.

The role of folate as an essential cofactor, coupled with the historical importance of the pathway in the development of antibacterial, antiparasitic, and anticancer therapies (Hoffbrand & Weir, 113(3) Brit. J. Haematol 579-89 (2001)), has led to folate metabolism being an especially well-characterized area of biology. The discovery of a novel, widely distributed folate biosynthetic enzyme, as described herein, illustrates the power of comparative genomic approaches to link genes and function. The signature genes of the *de novo* folate pathway, *folP* and *folK*, encode dihydropteroate synthase and 6-hydromethyl-7,8-dihydropterin pyrophosphokinase, respectively. All organisms that possess these two genes should have a homolog of the *folE* gene, because none of the metabolic intermediates from 7,8-dihydropterin triphosphate to 7,8-dihydro-hydroxymethylpterin pyrophosphate, are transported in bacteria. Skold, 3(3) Drug Resistance Update, 155-60 (2000).

The distribution of the *folE*/*folE2* genes among the sequenced organisms in the SEED database (26 archaeal, 363 bacterial, and 29 eukaryeal more or less complete genomes) were analyzed. No *FolE2* homolog was identified in the eukaryotic genomes, and there was significant variation in the distribution of the *FolE*/*FolE2* genes among bacteria. Analysis of the distribution of the *folE* gene among all sequenced genomes that possessed *folKP* homologs revealed a large class of organisms that lacked *folE* homologs (Table 1), suggesting that *folE* was "locally missing" in these organisms. *See also* Koonin et al., 12(9) Trends Genet. 334-36 (1996); Suppl. data for El Yacoubi et al., 2006, available at JBC Online.

**Table 1. Distribution of FolE, COG1469, FolK, and FolP homologs in a subset of sequenced genomes. The genomes used in the phylogenetic pattern search are in bold.**

| Organism | FolE | COG1469 | FolK | FolP |
|---|---|---|---|---|
| Escherichia coli K12 | + | | + | + |
| Bacillus subtilis subsp. subtilis str. 168 | + | + | + | + |
| Acinetobacter baylyi | + | + | + | + |
| **Bordatella bronchiseptica RB50** | | + | + | + |
| **Neisseria gonorrhoeae FA 1090** | | + | + | + |
| **Neisseria meningiditis MC58** | | + | + | + |
| **Neisseria mengingiditis FAM18** | | + | + | + |
| **Neisseria lactamica ST-640** | | + | + | + |
| **Nitrosomonas europaea ATC 19718** | | + | + | + |
| **Oceanobacillus iheyensis HTE831** | | + | + | + |
| **Staphylococcus aureus subsp. aureus MW2** | | + | + | + |
| **Staphylococcus haemolyticus JCSC1435** | | + | + | + |
| **Staphylococcus saprophyticus** | | + | + | + |
| **Thermotoga maritime MSB8** | | + | + | + |
| **Desulfotalea psychrophila LSv54** | | + | + | + |
| **Desulfuromonas acetoxidans** | | + | + | + |
| **Exiguobacterium sp. 255-15** | | + | + | + |
| **Geobacter sulferreducens PCA** | | + | + | + |
| **Geobacter metallireducens GS-15** | | + | + | + |
| **Magnetococcus sp. MC-1** | | + | + | + |
| **Methylobacillus flagellatus KT** | | + | + | + |
| **Silicobacter sp. TM1040[B]** | | + | + | + |
| **Rhodobacter sphaeroides 2.4.1** | | + | + | + |
| Idiomarina loihiensis L2TR | | + | + | + |
| Oceanicola batsensis HTCC2597 | | + | + | + |
| Rhodobacterales bacterium HTCC2654 | | + | + | + |
| Roseovarius nubinhibens ISM [B] | | + | + | + |
| Loktanella vestifoldensis SKA53 | | + | + | + |
| Thiomicrospira crunogena XCL-2 | | + | + | + |
| Sulfitobacter sp. EE-36 | | + | + | + |
| Roseobacter sp. MED193 | | + | + | + |
| **Desulfovibrio vulgaris** | | + | + | + |

Using a SEED tool that allows identification of protein families that follow a defined phylogenetic distribution profile, a search was conducted of the available genomes for protein families that were present in organisms that lack *folE* homologs (Table 1, in bold) and absent in *E. coli.* Five protein families fulfilled those phylogenetic criteria, one of which, COG1469, was of unknown function. Members of this family clustered physically with folate metabolism genes in several organisms. The combination of phylogenetic distribution and clustering suggested that eh COG1469 family might encode the missing GCYH-I enzyme.

Further regarding the variation of the distribution, the first and largest group, which includes *E. coli,* has only a *folE* homolog. A second group, which includes *S. aureus* and *N. gonorrhoeae,* has only a *folE2* homolog. A third group, including *B. subtilis* and *A. baylyi,* has a homolog of each gene, whereas a fourth group can possess multiple copies of the two genes (e.g. *Pseudomonas aeruginosa* has two *folE* genes and one *folE2* gene). The need for several genes encoding type I cyclohydrolase enzymes in many organisms may be due to differential expression under specific environmental conditions or their involvement in pathways other than folate biosynthesis: for example, a GTP cyclohydrolase has been implicated in the biosynthesis of 7-deazaguanosine derivatives, such as the modified tRNA nucleoside queuosine (Kuchino et al., 3 Nucl. Acids. Res. 393-98 (1976)), and the secondary metabolites toyocamycin and tubercidin. Suhadolnik & Uematsu, 245(17) J. Biol. Chem. 4365-71 (1970); Smulson & Suhadolnik, 242(12) J. Biol. Chem. 2872-76 (1967). In *B. subtilis,* it has been shown that the *yciA* gene is not essential (Gaballa et al., 184 J. Bacteriol. 6508-14 (2002)), because a folE gene (*mtrA*) is also present in this organism. Yakhin & Babitze, 64(3) Appl. Microbiol. Biotech. 382-86 (2004). No *folE2* deletions are available in bacteria that do not have another identified *folE* gene; but construction of the corresponding *S. aureus* mutant is possible.

Most archaeal genomes possess either a *folE* or a *folE2* homolog. *See* El Yacoubi et al., 2006, Suppl. Data. Several GTP-derived metabolites are synthesized in Archaea, including folate in the halophiles and *Sulfolobii* (White, 170(10) J. Bacteriol. 4608-12 (1988)), tetrahydromethanopterin in the methanogens (Graham et al., 41(50) Biochem. 15074-84 (2002)), and the 7-deazaguanosine tRNA-modified nucleoside archaeosine (Gregson et al., 268(14) J. Biol. Chem. 10076-86 (1993)), which is found in the majority of archaeal tRNA. The archaeal *folE*/*folE2* genes may be involved in one or more of these biosynthetic pathways.

As noted above, GCYH-IB has been identified in a wide variety of bacteria using the techniques described herein. Table 2 illustrates some of this distribution:

**Table 2. Distribution of genes encoding GCYH-1B in prokaryotes. Genome and gene ids are found in the SEED database.**

| Genome ID | Organism | GCYH-IB |
|---|---|---|
| 272557.1 | Aeropyrum pernix K1 [A] | 1635 |
| 224325.1 | Archaeoglobus fulgidus DSM 4304 [A] | 1168 |
| 64091.1 | Halobacterium sp. NRC-1 [A] | 1638 |
| 348780.3 | Nitrosamonas pharaonis DSM 2160 [A] | 1042 |
| 18420.1 | Methanothermobacter thermautotrophicus str. Delta H [A] | 1179 |
| 243232.1 | Methanocaldococcus jannaschii DSM 2661 [A] | 797 |
| 267377.1 | Methanococcus maripaludis S2 [A] | 34 |
| 259564.1 | Methanococcoides burtonii DSM 6242 [A] | 34 |
| 188937.1 | Methanosarcina acetivorans C2A [A] | 4403 |
| 269797.3 | Methanosarcina barkeri str. fusaro [A] | 1194 |
| 2208.1 | Methanosarcina barkeri [A] | 3612 |
| 192952.1 | Methanosarcina mazei Go1 [A] | 1222 |
| 190192.1 | Methanopyrus kandleri AV19 [A] | 483 |
| 272844.1 | Pyrococcus abyssi GE5 [A] | 362 |
| 18497.1 | Pyrococcus furiosus DSM 3638 [A] | 1881 |
| 69014.3 | Thermococcus kodakaraensis [A] | 1153 |
| 263820.1 | Picrophilus torridus DSM 9790 [A] | 1009 |
| 273116.1 | Thermoplasma volcanium GSS1 [A] | 1214 |
| 273075.1 | Thermoplasma acidophilum DSM 1728 [A] | 1117 |

Importantly, in addition to *S. aureus* and *N. gonorrhoeae,* there are significant pathogens that have GCYH-IB but do not have GCYH-IA, as shown in Table 3:

**Table 3. Clinically important pathogens with GCYH-IB and lacking GCYH-IA.**

| Organism | Pathogenic Indication |
|---|---|
| Bordetella parapertussis 12822 | Mammalian pathogen, respiratory tract infection |
| Bordetella parapertussis 12822 | |
| Bordetella pertussis Tohama I | |
| Neisseria gonorrhoeae FA 1090 | Human pathogen, genital and urinary tract infection |
| Neisseria meningitidis FAM18 | Human pathogen, meningitis |
| Neisseria meningitidis MC58 | |
| Neisseria meningitidis ZZ491 | |
| Staphylococcus aureus RF122 | Human pathogen, blood and skin infection, toxic shock syndrome |
| Staphylococcus aureus subsp. aureus COL; subsp. aureus JH1; subsp. aureus MRSA252; subsp. aureus MSSA476; subsp. aureus MW2; subsp. aureus Mu50; subsp. aureus N315; subsp. aureus NCTC 8325; subsp. aureus USA300; subsp. aureus | |
| Staphylococcus epidermidis ATCC 12228 | Human pathogen, blood, skin, and catheter infection |
| Staphylococcus epidermidis RP62A | |
| Staphylococcus haemolyticus JCSC1435 | Human pathogen, skin infection |
| Staphylococcus saprophyticus subsp. ATCC 15305 | Human pathogen, acute urinary tract infection |

The primary structure of GCYH-IB proteins presents no homology to any other known protein family. Direct alignment of GCYH-IB and GCYH-IA sequences yields no detectable similarity. Protein fold recognition analysis using 1- and 3-dimensional sequence profiles, however, coupled with secondary structure and solvation potential information (using the 3D-PSSM server available on-line from the Structural Bioinformatics Group at the Imperial College, UK; Kelley et al., 299(2) J. Mol. Biol. 499--520 (2000)), indicates potential three-dimensional structural homology with two tunnel-fold (T-fold) enzymes, a structural superfamily of enzymes that includes GCYH-IA. Colloc'h et al., 39(2) Proteins 142-54 (2000). T-fold enzymes bind planar purine and pterin-like substrates but catalyze disparate reactions (*id.*)*,* and although they characteristically exhibit low sequence homology, their tertiary structural homology is very high.

Using the *N. gonorrhoeae* sequence as a bait, the N-terminal half of GCYH-IB is most similar in predicted tertiary structure to 7,8-dihydroneopterin triphosphate epimerase (Protein Data Bank code 1B9L (44), PSSM E value 0.39), whereas the C-terminal half is similar to 7,8-dihydroneopterin aldolase (DHNA; Protein Data Bank code 1NBU (45), PSSM E value 0.3). These were the only PSSM hits with a qualifying E value (i.e., lower than the detection threshold E value of 1.00). Both hits are folate biosynthetic enzymes with homo-octameric structures. Both the size of GCYH-IB proteins (250-300 amino acids) and the fact that two T-fold domains can be detected in their sequences suggest that GCYH-IB members belong to the bimodular subfamily of the T-fold superfamily, which includes urate oxidase (Colloc'h et al., 4(11) Nat. Struct'l Biol. 947-52 (1997)), the plant GCYH-IA enzyme (Basset et al., 99(19) PNAS 12489-94 (2002)), and the novel nitrile oxidoreductase (class 2; e.g. YqcD from *E. coli)* recently reported. Van Lanen et al., 102(2) PNAS 4264-69 (2005). Preliminary sedimentation velocity and crystallographic analyses of *N. gonorrhoeae* GCYH-IB suggest either a trimeric or a tetrameric quaternary structure.

Of the enzymes involved in folate and biopterin biosynthesis, GCYH-IA has attracted particular attention (Nar et al., 1995; Tanaka et al., 138(3) J. Biochem. (Tokyo) 263-75 (2005); Schramek et al., 316(3) J. Mol. Biol. 829-38 (2002); Bracher et al., 273(43) J. Biol. Chem. 28132-141 (1998); Wolf & Brown, 192(3) Biochem. Biophys. Acta 468-78 (1969)), due to the mechanistic complexity inherent in the conversion of GTP to H2NTP. GCYH-IA activity is dependent on a catalytic Zn²⁺ atom (Auerbach et al., 97(25) PNAS 13567-72 (2000)), which functions as a Lewis acid in activating a water molecule for nucleophilic attack at C-8 of GTP in the initial hydrolytic step of the reaction. The Zn²⁺ further serves to facilitate nucleophilic attack of the second water molecule by polarizing the resulting amide carbonyl. The zinc-binding site in GCYH-IA is made up of Cys110, His113, and Cys181 (*E. coli* numbering), with water occupying the fourth coordination site.

Hence, GCYH-IB was discovered in microbes (20% of bacteria and most archaea) that do not encode the canonical GCYH-I (renamed GCYH-IA), including several clinically important pathogens such as *N. gonorrhoeae* and *S. aureus.* El Yacoubi et al., 2006. Importantly, GCYH-IB is absent in eukarya, including humans. A prediction of the 3D structure of GCYH-IB showed that, like GCYH-IA, GCYH-IB enzymes are members of the tunnel-fold (T-fold) structural superfamily. GCYH-IB enzymes from *T. maritima*, *N. gonorrhoeae,* and *B. subtilis* have been cloned and functionally characterized *in vitro.* The metal dependency of GCYH-IB was analyzed *in vitro* and was found to be distinct from that of GCYH-IA, with manganese as the preferred metal cofactor. The preference for manganese of the *N. gonorrhoeae* enzyme is consistent with the fact that this pathogen had evolved unique and complex manganese-based cellular mechanisms for coping with the high oxidative stress environment imposed on it by the innate immune response of the female urogenital tract. Seib et al., 70(2) Bio. Reviews 344-61 (2006). The primary defenses used by *N. gonorrhoeae* against oxidative stress include the intracellular accumulation of manganese by the MntABC transport system and the unusually high manganese-dependent catalase and peroxidase activities. The discovery of a manganese-dependent folate biosynthesis enzyme in *N. gonorrhoeae* paves the way to a new approach in targeting the folate biosynthesis pathway for the development of anti-gonorrhea antibiotics.

The present invention provides for the crystal structures of *N. gonorrhoeae* GCYH-IB and of the manganese-remetallated form of the enzyme according to claim 1. An aspect of the present invention reveals the enyzme's active center, including a metal binding site, which are distinct from those of the canonical enzyme. The structural differences between human GCYH-IA and bacterial GCYH-IB in the active center, including those in the metal binding site, suggest distinct enzymatic mechanisms. These differences offer the unique opportunity to design and test inhibitors specific to the bacterial enzyme (GCYH-IB) that will not inhibit the human enzyme.

As noted above, the Zn²⁺-dependent enzyme GTP cyclohydrolase I (GCYH-I; EC 3.5.4.16) is the first enzyme of the de novo tetrahydrofolate (THF) biosynthesis pathway (Fig. 1). Nichol et al., 54 Ann. Rev. Biochem. 729-64 (1985). THF is an essential cofactor in one-carbon transfer reactions in the synthesis of purines, thymidylate, pantothenate, glycine, serine, and methionine in all kingdoms of life (*id*.) and formylmethionyl-tRNA in bacteria. Clark & Marcker, 17 J. Mol. Biol. 394-406 (1966). GCYH-I is encoded in *E. coli* by the *folE* gene (Katzenmeier et al., 372 Biol Chem. Hoppe Seyler 991-97 (1991)), and catalyzes the conversion of GTP to 7,8-dihydroneopterin triphosphate, a complex reaction that begins with hydrolytic opening of the purine ring at C-8 of GTP to generate an N-formyl intermediate, followed by deformylation and subsequent rearrangement and cyclization of the ribosyl moiety to generate the pterin ring in THF (Fig. 1). Yim & Brown, 251 J. Biol. Chem. 5087-94 (1976). An active-site Zn²⁺ activates a water molecule for nucleophilic attack at C-8 in the first step of the reaction.

The distribution of *folE* (gene product renamed GCYH-IA) and *folE2* (GCYH-IB) in microbes is diverse. El Yacoubi et al, 326 J. Mol. Biol. 503-516 (2006). The majority of organisms possess either a *folE* (65%, e.g., *E. coli)* or a *folE2* gene (14%, e.g., *N. gonorrhoeae*). A significant number (12%, e.g., *B. subtilis)* possess both genes (a subset of 50 bacterial species is shown in Fig. 13), and 9% lack both genes, although members of the latter group are mainly intracellular or symbiotic bacteria that rely on external sources of folate.

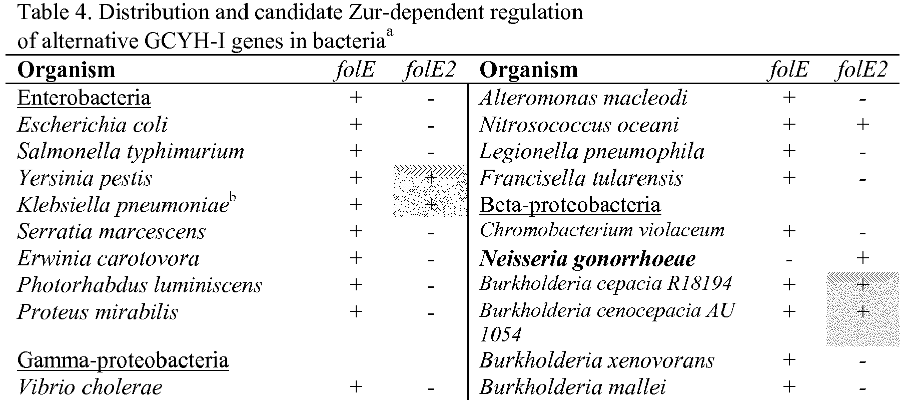

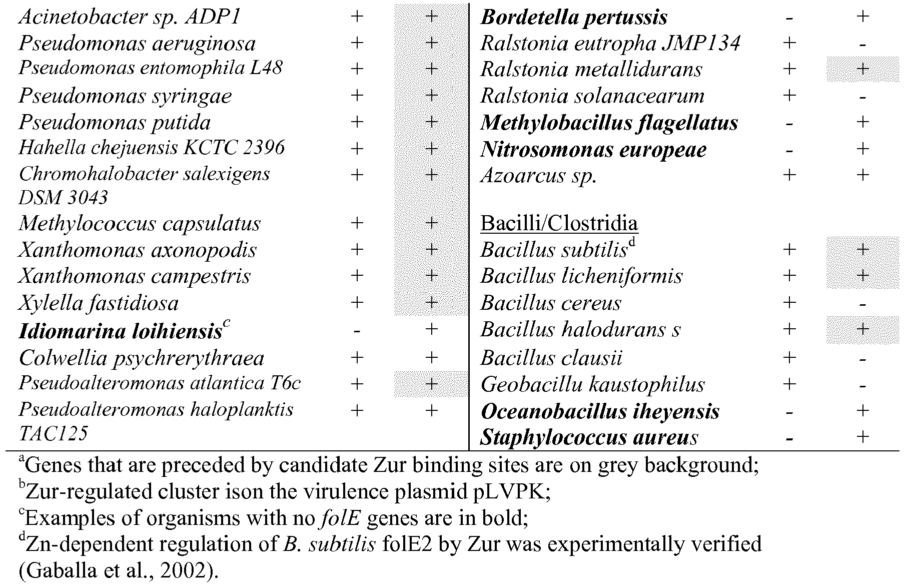

Expression of the *folE2 B. subtilis* gene, *yciA*, is controlled by the Zn-dependent Zur repressor and should thus be up-regulated under Zn-limiting conditions. Gaballa et al., 184 J. Bacteriol. 6508-14 (2002). Hence, the GCYH-IB family might utilize a metal other than Zn to allow growth in Zn-limiting environments. The metal dependence *of B. subtilis* GCYH-IB *in vitro* and *in vivo* was explored, revealing that that in organisms that contain both isozymes such as *B. subtilis,* only the Zn-dependent enzyme is expressed unless Zn becomes limiting. To gain a structural understanding of the metal dependence of GCYH-IB, the high-resolution crystal structures of Zn²⁺- and Mn²⁺-metallated forms of the *N. gonorrhoeae* ortholog were determined. The results shed light on the regulation of folate biosynthesis in organisms exposed to different metal environments and offer a structural understanding of this regulation.

GCYH-IB and GCYH-IA have different metal requirements *in vitro.* It is well established that GCYH-IA uses a bound Zn²⁺ ion for activity. Nichol et al., 1985. To investigate the metal dependence of GCYH-IB, the purified recombinant *B. subtilis* enzyme was assayed for activity in the presence of a variety of metal ions or EDTA. Although no activity was observed in the presence of EDTA, the presence of several metal ions supported catalysis. To obtain unambiguous, quantitative data on the effect of various metal ions on catalytic activity, the enzyme was first demetallated by dialyzing against an EDTA/Chelex-containing buffer to generate the apoenzyme, which was then assayed in the presence of specific metal ions over a broad concentration range. As shown in Table 5, catalysis is supported by the metal ions Mn²⁺, Fe²⁺, Mg²⁺, Co²⁺, Zn2⁺, and Ni²⁺, conversely Ca²⁺, Cd²⁺, Cu²⁺, Co³⁺, and Fe³⁺ fail to support activity:

**Table 5 Metal dependence of B. subtilis GCYH-IB enzymatic activity**

| Metal | [Metal]^{a} (µM) | Relative Activity (%) |
|---|---|---|
| No Metal | 0 | 0 |
| Mn(II) | 500 | 100 |
| Fe(II) | 1000 | 75 ± 8 |
| Mg(II) | 100 | 43 ± 4 |
| Co(II) | 100 | 24 ± 3 |
| Zn(II) | 50 | 14 ± 1 |
| Ni(II) | 100 | 9.8 ± 1.3 |
| Ca(II) | NA^{b} | 0 |
| Cd(II) | NA | 0 |
| Cu(II) | NA | 0 |
| Co(III) | NA | 0 |
| Fe(III) | NA | 0 |

| | | |
|---|---|---|
| ^{a}The metal concentration for optimal activity. ^{b}NA refers to no activity detected regardless of metal concentration. | | |

Notably, although the enzyme exhibits some activity in the presence of Zn²⁺, catalysis is significantly higher in the presence of Mn²⁺, and to a lesser extent Fe²⁺ and Mg²⁺. Interestingly, the optimum metal concentration required for catalysis is roughly 10-fold lower for Zn²⁺ than it is for Mn²⁺ (Fig. 10, Table 5, suggesting that although Mn²⁺ is more effective in supporting catalysis, it binds with lower affinity than does Zn²⁺.

The presence of both Zn²⁺ and Mn²⁺ in a reaction assay results in diminished activity (i.e. Zn²⁺ is an inhibitor versus Mn²⁺, Table 6). All of the metals bind the enzymes with low affinity, such that running the protein over a G-25 column or dialyzing against metal free buffer removed the protein bound metal.

**Table 6. The effect of zinc upon B. subtilis and N. gonorrhoeae GCYH-IB activity when assayed in the presence of 600µM Mn(II). Data expressed as relative activity (%).**

| [zinc] µM | *B. subtilis* | *N. gonorrhoeae* |
|---|---|---|
| 0 | 100 | 100 |
| 0.5 | 84 | 93 |
| 1 | 41 | 68 |
| 2 | 34 | 57 |
| 4 | 35 | 47 |
| 10 | 35 | 53 |
| 20 | 26 | 45 |
| 40 | 23 | 45 |

To understand the structural basis of the unique metal requirement of GCYH-IB, the crystal structure in two forms - one of the recombinant enzyme purified in solutions lacking added metal (GCYH-IB, PDB ID 3D1T), and one of the apoenzyme remetallated with manganese (GCYH-IB•Mn, PDB ID 3D2O) - were determined. Enzymes from both *B. subtilis* and *N. gonorrhoeae* were pursued for crystallization, but only the *N. gonorrhoeae* ortholog produced diffracting crystals. The two orthologs are 64% similar and 35% identical in sequence and possess similar biochemical properties *in vitro.*

The crystal structure of GCYH-IB was determined by seleno-MAD methods (Fig. 2, Tables 7 and 9). Two monomers, A and B, were identified in the asymmetric unit. The final structure is missing residues 1-13 of monomer A and residues 1-14 of monomer B of the total 257 amino acids in each monomer, and contains two Zn²⁺ ions and one acetate molecule. A homotetrameric complex representing the biological state of the enzyme (El Yacoubi et al., 281 J. Biol. Chem. 37586-93 (2006)), could be generated from the asymmetric-unit dimer by a crystallographic 2-fold rotation operation (Fig. 2A). (32% of the total monomeric surface is buried in the homotetramer). The monomer is a globular subunit composed of 9 β-strands and 6 α-helices (Fig. 3A), of which 8 sequential antiparallel β-strands (β1- β6, β8 and β9) and four antiparallel α-helices (α1, α2, α4 and α6) form a core with the classic tunneling fold (T-fold) architecture characteristic of bimodular pterin and purine binding enzymes. Colloc'h et al., 39 Proteins 142-54 (2000). In this core, a highly twisted eight-stranded β-sheet is layered on its concave side with four antiparallel α-helices. The dimer of the asymmetric unit is constructed by a cyclic arrangement of the two eight-stranded β-sheets from the two monomers to form a sixteen-stranded antiparallel β-barrel. In the biological homotetramer, two β-barrels join together head-to-head to form a central tunnel that is 60Å long and 17 Å in diameter (Fig. 2A).

A search for similar structures was done using the DALI search engine and the FSSP database (fold classification based on structure-structure alignment of proteins). Holm & Sander 233 J. Mol. Biol. 123-38 (1993). Several bimodular T-fold enzymes were identified, as well as GCYH-IA (Fig. 3C), a unimodular T-fold enzyme. The best fit was to *A. flavus* urate oxidase (Retailleau et al., 60 Acta Crystallogr. D 453-62 (2004)) (PDB ID 1UOX, r.m.s.d. 3.4 Å over 197 C_{α} atoms, Fig. 3B) that follows a similar β8α4 topology.

Comparison of GCYH-IA and GCYH-IB structures shows that GCYH-IB is a homotetramer built around a bimodular β8α4 T-fold core and GCYH-IA is a homodecamer β4α2 unimodular T-fold enzyme. Pairwise structural comparison of the monomeric subunits of GCYH-IB and *E. coli* GCYH-IA (Rebelo et al., 326 J. Mol. Biol. 503-16 (2003)) (PDB ID 1FBX) using the DaliLite server (Holm & Park, 16 Bioinform. 566-67 (2000)), yielded an alignment strictly in the four β-strands and two α-helices (β2, β3, β6, β7, α6, α7 of GCYH-IA, and β5, β6, β8, β9, α4, α6 of GCYH-IB) of the T-fold (r.m.s.d. 3.2 Å over 94 C_{α} atoms, Fig. 3C). Based on this alignment, a multi-sequence alignment of the two enzyme subfamilies in the shared core region was generated (15 sequences of each, Fig. 4). With the exception of a conserved cysteine involved in metal binding in both subfamilies (Cys147 in GCYH-IB), the new alignment differs significantly from previously reported alignment that was based on sequence information and predicted tertiary structure.

In the current alignment, the two subfamilies exhibit 28% sequence similarity and only two invariant residues; the metal and substrate liganding residues Cys147 and Glu216 *(N. gonorrhoeae* numbers), respectively. This unusually low primary structure homology, compared with 7%-17% identity and 39%-53% similarity between T-fold enzymes in general, explains improper annotation of GCYH-IB genes in genomic databases and limited success in previous attempts to generate a model of the tertiary structure by sequence-based homology modeling (El Yacoubi et al, 2006).

In addition to the absence in GCYH-IB of the N- and C-terminal domains found in GCYH-IA (Figs. 3A and 3C), the following functionally important differences are seen in the T-fold domain, specifically in the active site: (a) insertion in GCYH-IB of a two turn α-helix (α3) between the first two strands (β5 and β6) of the common T-fold β-sheet (Figs. 3A, 3C and Fig. 4). This change results in the loss of the Zn²⁺ binding loop C110EHH113 found in GCYH-IA, with the exception of the Cys (Fig. 4); (b) Insertion in GCYH-IB of a β-strand (β7) and α-helix (α5) between the two α-helices (α4 and α6) of the T-fold (Figs. 3A, 3C and Fig. 4). β7 and α5 are part of the inter-subunit interface that harbors the active site. Significantly, α5 provides the strictly conserved Glu201 as a carboxylate ligand to the active-site metal ion in GCYH-IB; and (c) Deletion in GCYH-IB of helix α8 found in the T-fold domain of GCYH-IA (Figs. 3A, 3C, and Fig. 4). Importantly, α8 contains the second Cys ligand to Zn²⁺ in GCYH-IA. This Cys is missing in GCYH-IB and the helix is replaced with a β-turn containing strictly conserved Glu243, His246 and His248.

Rearranged metal binding site and accommodation of Mn²⁺ as in GCYH-IA, the active site of GCYH-IB is located at the interface between three subunits. Two of the four active sites in the GCYH-IB homotetramer are partially disordered in the crystal. The active site encompasses the metal binding site and the putative GTP binding pocket with the conserved Glu216 that serves to anchor the substrate guanine moiety, a characteristic feature of all T-fold proteins (Colloc'h et al, 2000).

The presence of bound Zn²⁺ in the GCYH-IB crystal (grown with no additional Zn in the crystallization buffer) was confirmed by an X-ray fluorescence scan near the Zn absorption edge (see Fig. 12A) and an omit Fo-Fc map (Fig. 5A). Zn²⁺ is bound in a trigonal bipyramidal geometry and is coordinated by the thiol group of Cys 147, side-chain oxygen atom of Glu201 from the neighboring subunit, and an oxygen atom from a bound acetate molecule (present in the protein sample at 50 mM) as equatorial ligands, and Nεof His159 and a water molecule as the bottom and top axial ligands, respectively (Figs. 5A and 6). The average Zn²⁺-to-ligand distance is 2.4 Å. In its current position, the acetate molecule may mimic one of the cyclohydrolase reaction transition states that are subsequent of the opening of the substrate guanosine ring. The liganding side chains, their positions in the Zn²⁺ coordination sphere and the protein secondary structure elements in which they lie are different from those seen in GCYH-IA (Figs. 4, 6). In *T. thermophilus* and *E. coli* GCYH-IA (PDB-ID 1WM9, Rebelo et al., 326 J. Mol. Biol. 503-516 (2003); Tanaka et al., 138 J. Biochem. 263-275 (2005)), Zn²⁺ is coordinated by two thiol groups and a histidine side chain (Cys110, Cys181, His113 in *E. coli* residue numbers), all from the same subunit. These side chains come from the loop between the first two β-strands of the T-fold (β2 and β3 in Ec-GCYH-IA nomenclature) and a short α-helix (α8) (Fig. 4). In GCYH-IB, the metal binding site is formed by the helix-loop insertion (α3) between the first two β strands of the T-fold (β5 and β6) and the helical insertion α5 from the neighboring subunit.

Crystals of GCYH-IB remetallated with Mn²⁺ (GCYH-IB•Mn) were grown using Zn-free reagents. The presence of bound Mn²⁺ was confirmed with an X-ray emission spectrum and fluorescence scan near the Mn absorption edge following a wash and 1-hr back-soak of the crystal in metal-free solution (see Figs. 12B and 12C). The crystal structure was determined by difference Fourier methods using model phases from the Zn²⁺-metallated structure (Tables 7 and 9). The two structures are similar and superpose with r.m.s.d. 0.3 Å over 486 Caatoms. Metal coordination and distances are also similar except for the replacement of acetate with azide (added to the acetate-free protein sample at 12 mM concentration prior to crystallization) as an equatorial ligand to Mn²⁺ (Fig. 5B).

The Zur dependent regulation of *folE2* is conserved across bacterial species. The biochemical and structural analyses *of B. subtilis* and *N. gonorrhoeae* GCYH-IB suggest that Zn is not the physiological metal for this family of cyclohydrolases. The expression of GCYH-IB in *B. subtilis* is controlled by the Zn-dependent Zur repressor and is thus up-regulated under Zn-limiting conditions. Gelfand et al., 1 Brief Bioinform 357-71 (2000). To check if orthologs of *folE2* in other bacteria are similarly subject to Zur-mediated control, bacterial regulons were analyzed using a comparative genomics approach. Gelfand et al, 2000; Rodionov, 107 Chem. Rev. 3467-97 (2007). Annotation of the *folE* and *folE2* genes in available genomes had been performed previously. Two position-specific weight matrices (PWMs) were constructed for known Zur binding sites from Gram-positive and Gram-negative bacteria (Panina et al., 100 Proc Natl Acad Sci U S A 9912-17 (2003)), and were used to scan for candidate Zur binding sites in 5'-untranslated regions (UTRs) of *folE2* and neighbor genes in bacterial genomes. To account for possible operon structures, UTRs of genes located immediately upstream of *folE2* in the genomes were also analyzed.

Strong Zur operator sites were identified upstream of folE2 genes in twenty-one bacterial genomes (Table 4, Fig. 13). This unique regulatory feature is conserved across phyla including examples in fifteen γ-proteobacteria (*Pseudomonadales, Xanthomonadales,* some *Enterobacteria,* and other lineages), three β-proteobacteria (some *Burkholderiales*), and three *Bacillus* species. Notably, all organisms that have the Zur-regulated *folE2* gene also contain a copy *offolE* (e.g., *B. subtilis*)*.* Similarly, no Zur regulatory sites were found upstream of *folE2* genes in those organisms that lack the Zn-dependent GCYH-IA isozyme (e.g., *N. gonorrhoeae*). These results show that Zur regulation of GCYH-IB is widespread and suggest that, in some bacteria, the GCYH-IB isozyme is expressed under Zn-limiting conditions to replace the Zn-dependent GCYH-IA.

GCYH-IB can functionally replace GCYH-IA in *B. subtilis,* which has both the *folE* and *folE2* genes (also known as *mtrA* and *yciA*, respectively). To determine whether GCYH-IB can functionally replace GCYH-IA, a *B. subtilis* Δ*folE* mutant strain was constructed and its complementation with GCYH-IB investigated. Wild-type *B. subtilis* grows well in rich (LB) medium (Fig. 11, diamonds). As reported previously (Babitzke et al., 174 J. Bacteriol. 2059-64 (1992)), the Δ*folE* mutant strain, expressing only GCYH-IB, is a thymidine (dT) auxotroph. In the absence of dT, it grows only with a reproducible lag in rich medium (Fig. 11, squares). Poor growth of the Δ*folE* mutant strain in log phase is consistent with repression of GCYH-IB expression under these conditions. Growth commences in stationary phase and reflects derepression of GCYH-IB when cells become Zn-starved and Zur repression is released. Indeed, deleting the zur gene, as in the Δ*zur* Δ*folE* double mutant where GCYH-IB is constitutively expressed, eliminates the growth lag in rich medium (Fig. 11, triangles). As expected, the Δ*folE* Δ*zur* Δ*folE2* triple mutant strain, expressing no GCYH-I, is unable to grow (Fig. 11, closed circles), nor is the Δ*folE* Δ*folE2* double mutant strain. For either strain, growth can only be partially restored by the addition of dT (Fig. 11, open circles) or all of the folate-derived metabolites. The rather poor dT rescue of these two mutant strains may be due to deficient formylated methionine biosynthesis. These results indicate that GCYH-IB functionally replaces GCYH-IA in *B. subtilis* under Zn-limiting conditions.

Sequence conservation indicates that metal-dependent catalysis is a property of the GCYH-IB enzyme family in general, with Mn²⁺, not Zn²⁺, possibly the preferred metal *in vitro.* Indeed, metal catalysis has been observed with GCYH-IB orthologs from *N. gonorrhoeae* and *T. maritima*, and from *M. jannaschii* (Grochowski et al., 46 Biochem. 6658-67 (2007)), which exhibits optimal activity in the presence of Fe²⁺. The structural differences between GCYH-IA and -IB enzymes in the active-site region contribute to creating a new metal functionality in GCYH-IB. For example, the introduction of an acidic side chain (Glu201) in the metal site of GCYH-IB allows accommodation of Mn²⁺ which favors oxygen ligands more than Zn²⁺. Further, the contribution of a neighboring subunit to the metal site suggests possible posttranslational regulation by metal-induced oligomerization as seen in DHNA. Goulding et al., 349 J. Mol. Biol. 61-72 (2005). The metal ion likely serves to activate the nucleophile water molecule in the first step of the reaction and to stabilize the formyl intermediate, as in GCYH-IA. Tanaka et al, 2005.

The differences in the active-site architecture and associated metal dependence between GCYH-IA and -IB may reflect differences in catalytic strategies for the two enzyme families. The presence of acetate and azide as exogenous metal ligands in the Zn²⁺- and Mn²⁺-metallated enzyme structures, respectively, is a result of sample preparation conditions and does not reflect specificity to the type of metal. The acetate ligand may mimic the reaction formyl intermediate or formic acid as a leaving group. The azide ligand may represent a moiety in a transition-state intermediate occurring in a later step, e.g., during the Amadori rearrangement that takes place after guanine and ribose ring opening.

Zinc is an essential cofactor for numerous proteins. In Bacteria and Eukaryotes, cellular Zn²⁺ levels are sensed by specific transcription factors (the repressor Zur in *B. subtilis* and *E. coli,* the activator Zaplp in *S. cerevisiae*). Hantke, 8 Curr. Opin. Microbiol. 196-202 (2005); Lyons et al., 97 Proc. Nat'l Acad. Sci. USA 7957-62 (2000); Moore & Helmann, 8 Curr. Opin. Microbiol. 188-195 (2005). Cellular responses to low Zn²⁺ conditions include increased expression of high-affinity Zn²⁺ transporters such as ZnuABC, and substitution of Zn²⁺-dependent enzymes with alternative isozymes that do not rely on this metal ion as a cofactor. Examples of the latter strategy include Zn²⁺-dependent regulation of the alcohol dehydrogenase isozyme ADH4 in yeast (Lyons et al., 2000), and paralogs ofribosomal proteins (e.g., L31 and L33) in *B. subtilis* and *Streptomyces coelicolor.* Nanamiya et al., 52 Mol. Microbiol. 273-83 (2004); Panina et al, 2003; Shin et al., 189 J. Bacteriol. 4070-77 (2007).

Similarly, results from the work presented herein indicate that the upregulation *of B. subtilis* GCYH-IB when Zn²⁺ is low serves to allow utilization of metal ions other than Zn²⁺ for folate synthesis. On the other hand, *N. gonorrhoeae* (and several other pathogens) do not possess the Zn-dependent GCYH-IA isozyme and instead rely solely on GCYH-IB for folate synthesis. Consistently, they do not have Zur regulatory sites upstream of the *folE2* gene. *N. gonorrhoeae* live in the highly oxidizing environment created by the host immune response and, to survive in this challenging environment, they accumulate millimolar levels of manganese, an effective scavenger of reactive oxygen species (ROS), in the cytoplasm. Seib et al., 70 Microbiol. Mol. Biol. Rev. 344-61 (2006). A similar utilization of Mn to fight ROS damage resulting from radiation exposure occurs in *D. radiodurans.* Daly et al., 306 Science 1025-28 (2004). Presumably, the ready availability of Mn²⁺ makes a Mn-dependent folate biosynthesis pathway more advantageous for these organisms.

Further, a recent report shows that the innate immune system in mice fights *S. aureus* infections by inhibiting microbial growth in tissue abscesses through chelation of Mn²⁺ and Zn²⁺ by the neutrophil-derived protein calprotectin, depriving the bacteria of essential nutrients. Corbin et al., 319 Science 962-65 (2008). It is possible that this defense strategy targets, among other cellular processes, folate synthesis in S. aureus, an organism that depends solely on GCYH-IB for folate synthesis and lacks GCYH-IA (Fig. 13). Hence, the distinct structure and metal dependence of GCYH-IB and its absence in humans may make it an attractive target for the development of new antibiotic agents.

### EXAMPLES

### Example 1. Bioinformatics.

Analysis of the folate subsystem was performed in the SEED data base (Overbeek et al., 33(17) Nucl. Acids Res. 5691-5702 (2005)) with SEED version cvs.1144925141 (05:45:41 on April 13, 2006) (available on the internet at, for example, the ".org" site of the National Microbial Pathogen Data Resource (NMPDR). Results are made available in the "Folate Biosynthesis Subsystem" on the publicly available server (available on the internet at the SEED cite of the University of Chicago). The phylogenetic pattern search was performed on the SEED server located on the NMPDR website.

### Example 2. Cloning of Thermotoga maritima, Neisseria gonorrhoeae, and Bacillus subtilis COG1469 genes for GCYH-IB protein expression.

The COG1469 genes from *T. maritima* (TM0039; GenBank® accession number gi|15642814), *N. gonorrhoeae* (ngo0387; GenBank® accession number gi|59800831), and *B. subtilis* (yciA; GenBank® accession number gi|2632620) were amplified by PCR from genomic DNA of the respective organisms. The primers have been included in Ser. No. 60/935,124 and published in El Yacoubi et al., 2006.

The PCRs contained 500 ng of genomic DNA, 200 µM dNTPs, 50 pmol of the sense and antisense primers, 1x Pfu Ultra buffer (supplied by the manufacturer), and 2.5 units of Pfu Ultra DNA polymerase in a final volume of 50 µl. A three-step PCR thermocycling protocol was utilized: (1) 94°C for 1 min; (2) 30 cycles of denaturation at 94°C for 1 min, annealing at 50°C for 2 min, and extension at 72°C for 1 min; (3) 72°C for 4 min. The PCR product was purified from a 1% agarose gel containing ethidium bromide using the Qiagen Inc. PCR purification kit and cloned into a linearized pET-30 Xa/LIC expression vector (Novagen). The primary structures of the resulting constructs, pSAB-7-189 (*T. maritima*), pSAB-8-142 (*N. gonorrhoeae*), and pSAB-9-61 (*B. subtilis*), were confirmed by sequencing.

### Example 3. Cloning of the T. maritima, Acinetobacter baylyi, and B. subtilis COG1469 genes for complementation.

pTM0039 expressing the TM0039 gene under PBAD control (Klock et al., 6(2-3) J. Struct. Genomics 89-94 (2005)) was a kind gift of the Joint Center for Structural Genomics (La Jolla, CA). The COG1469 genes from *B. subtilis* (yciA) and *A. baylyi* (ACIAD1740 gi|50084892) were cloned in pBAD24. Guzman et al., 177(14) J. Bact. 4121-30 (1995). The primers have been included in Ser. No. 60/935,124 and published in El Yacoubi et al., 2006. PCR products were obtained and purified as described above and then digested with NcoI/XbaI before ligation into plasmid pBAD24 (Guzman et al., 1995) digested with the same endonucleases and transformed into Topo10 cells (Invitrogen). The primary structures of the resulting constructs, pBY142.1 (expressing *A. baylyi* ACIAD1740) and pBY143.1 (expressing *B. subtilis* yciA), were confirmed by sequencing.

These plasmids, as well as pBAD24 and pTM0039, were transformed into the *E. coli folE*::KanR strain. Klaus et al., 280(46) J. Biol. Chem. 38457-63 (2005). The transformants were plated on LB supplemented with dT, ampicillin and kanamycin, and screened for the capacity to grow on LB without dT in the presence of various concentrations of arabinose. To confirm the presence of the *folE*::Kan^{R} allele and of the pBAD derivatives in the transformants, the colonies were analyzed by PCR using the oligonucleotides located upstream and downstream from the *folE* gene and ChkDfolE-ol2 or located upstream and downstream of the polylinker in the pBAD derivatives and pBADol5 as described in Ser. No. 60/935,124 and published in El Yacoubi et al., 2006.

### Example 4. Purification and overexpression of recombinant T. maritima, N. gonorrhoeae, and B. subtilis GCYH-IB proteins.

The plasmids pSAB-7-189, pSAB-8-142, and pSAB-9-61 were transformed into *E. coli* BL21 (DE3) for expression of His6 tag fusion proteins. Cultures of the transformed cells were grown at 37°C with shaking (250 rpm) until an A600 of 0.9 was attained. Isopropyl-D-thiogalactopyranoside was added to a final concentration of 0.1 mM, and the cultures were incubated for an additional 4 h at 37°C with shaking (250 rpm). The cells were harvested by centrifugation at 5000 x g for 10 min at 4°C. The cell paste was flash frozen in liquid nitrogen and stored at -80°C until needed.

Frozen cell paste was thawed and suspended in lysis buffer (50 mM Tris acetate (pH 8.0), 50 mM KCl, and 1 mM -mercaptoethanol) at a concentration of 250 mg/ml. The cells were lysed by the addition of lysozyme and DNase to a final concentration of 0.25 mg/ml and 10 µg/ml, respectively. The lysate was centrifuged at 15,000 x g for 30 min at 4°C, and the resulting supernatant was filtered (low protein binding, 0.45 µm). The cell-free extract was loaded onto an Ni²⁺-nitrilotriacetic acid-agarose column (Qiagen) that had been equilibrated with Buffer A (100 mM Tris-acetate (pH 8.0), 300 mM KCl, 2 mM β-mercaptoethanol, 1% Triton X-100, 1 mM phenylmethylsulfonyl fluoride, and 10% glycerol). The column was washed with five column volumes of Buffer A, five column volumes of Buffer B (100 mM Tris acetate (pH 8.0), 300 mM KCl, 2 mM β-mercaptoethanol, 1% Triton X-100, 1 mM phenylmethylsulfonyl fluoride, 10% glycerol, and 20 mM imidazole), and finally five column volumes of Buffer C (100 mM Tris acetate (pH 8.0), 300 mM KCl, 2 mM - mercaptoethanol, 10% glycerol, and 20 mM imidazole). The protein was eluted from the column with ten column volumes of Buffer C containing 250 mM imidazole. The protein was concentrated in a Centricon YM-10 ultracentrifugation device and dialyzed at 4°C against 50 mM Tris acetate (pH 8.0), 50 mM KCl, and 4 mM dithiothreitol.

The His6 tag was cleaved from the *T. maritima*, *N. gonorrhoeae,* and *B. subtilis* GCYH-IB proteins in reactions that contained fusion protein (20 mg), Factor Xa protease (20 µg), 50 mM Tris acetate (pH 8.0), 100 mM KCl, 2 mM CaCl₂ in a final volume of 1 ml. After incubating for 20 h at room temperature, the reactions were loaded onto a column containing 2 ml of Ni2+-nitrilotriacetic acid-agarose equilibrated in Buffer A. Wild-type protein was eluted from the column with ten column volumes of Buffer A. The protein was concentrated and dialyzed against 50 mM Tris acetate (pH 8.0), 50 mM KCl, and 10% glycerol.

### Example 5. COG1469 genes complement an E. coli folE mutant.

Because folate is not transported in most bacteria (Skold et al., 2000), it can not be supplied in the medium to enable growth of a folate auxotroph. On rich medium, however, all of the folate-derived metabolites are present in sufficient quantities except for dT, allowing a *folE* mutant to be maintained on LB/dT. Klaus et al., 2005; Yakhin & Babitzke, 2004. Nevertheless, the *E. coli folE*::KanR strain has a slow growth phenotype on LB/dT (colonies take two days instead of one day to form at 37°C), presumably due to the absence of formylation of the initiator tRNA. The *folE*::KanR strain was transformed with pBAD derivatives expressing the COG1469 homolog from *T. maritime* (TM0039).

Although complementation of both the dT auxotrophy and the slow growth phenotype was observed (Fig. 8C), was not robust, and depended on high arabinose levels. This is not surprising because *T. maritima* is a thermophile, and many of the enzymes from thermophiles exhibit low activity at 37°C. To achieve better complementation, the COG1469 orthologs from the mesophiles *B. subtilis* and *A. baylyi* (formally known as *Acinetobacter* sp. ADP1) were cloned and transformed into the *E. coli folE*::KanR strain. Robust complementation of dT auxotrophy (Fig. 8A) and poor growth (Fig. 8B) were observed with these constructs, which is consistent with GCYH-IB family proteins catalyzing GCYH-I activity.

### Example 6. GCYH-IB proteins have GCYH-I activity in vitro.

In parallel with the *in vivo* experiments, COG1469 genes were cloned into protein expression vectors to allow unambiguous assignment of catalytic function through the direct investigation of putative GTP cyclohydrolase I activity with in vitro enzymatic assays of purified proteins. Thus, the genes encoding GCYH-IB proteins from *T. maritima*, *N. gonorrhoeae,* and *B. subtilis* were cloned from genomic DNA into the pET30 system, and the recombinant His6 fusion proteins were overproduced and purified. All three of the recombinant proteins were obtained as soluble, active enzymes both as the His6 fusion and the cleaved wild type.

Radiochemical assays using [8-¹⁴C]GTP (Yim et al., 1976), of each of the GCYH-IB proteins, along with *E. coli* FolE as a positive control, demonstrated that [¹⁴C] formate was released in each assay and that its production was both time- and enzyme-dependent, consistent with enzyme-catalyzed hydrolytic ring opening and deformylation at C-8 of GTP. From these data, specific activities of 2.3 min⁻¹mg⁻¹ to 5.3 nmol min⁻¹mg⁻¹ were calculated for the GCYH-IB proteins, roughly an order of magnitude lower than that reported for *FolE* (Bracher et al., 274 J. Biol. Chem. 16727-35 (1999); Kolinsky & Gross, 279(39) J. Biol. Chem. 40677-82 (2004); Rebelo et al., 326(2) J. Mol. Biol. 503-16 (2003)) and the *FolE* control. To confirm that the product of the GCYH-IB catalyzed reactions was in fact 7,8-dihydroneopterin triphosphate, the enzyme assays were analyzed with UV-visible (Bracher et al., 40(26) Biochem. 7896-902 (2001)) and fluorescence spectroscopy. (Hatakeyama & Yoneyama, 100 Methods Mol. Biol. 265-72 (1998)). Fig. 9A shows UV-visible spectra for enzyme assays *of E. coli* FolE and GCYH-IB proteins under standard GTP cyclohydrolase I assay conditions. The spectra are essentially identical, with the characteristic absorption spectrum of GTP replaced by that of H2NTP. Bracher et al., 2001. When enzyme assays were subjected to post-reaction dephosphorylation and oxidation to convert the putative enzymatically produced H2NTP to the fluorescent neopterin, the fluorescent spectra from the GCYH-IB assays were identical to the spectrum of the *E. coli* FolE assay (Fig. 9B) and to that of authentic neopterin. Hatakeyama & Yoneyama, 1998.

Furthermore, HPLC analysis of the enzyme assays after dephosphorylation showed that the product from each enzyme-catalyzed reaction had the same retention time as authentic neopterin (under the analysis conditions dihydroneopterin is oxidized to neopterin) (Fig. 9C). Finally, mass spectrometry analysis of the *E. coli* FolE and *N. gonorrhoeae* GCYH-IB reactions revealed identical constituents, with ions corresponding to GTP *(m*/*z*522, M - H⁻; *m*/*z*544, M - 2H⁻ + Na⁺; *m*/*z*566, M - 3H⁻ + 2Na⁺), neopterin triphosphate (*m*/*z*492, M - H⁻; under the conditions of the analysis dihydroneopterin is oxidized to neopterin), and neopterin cyclic monophosphate (*m*/*z*314, M - H⁻; it has been previously documented that under the alkaline conditions of the work-up neopterin triphosphate is converted to the cyclic monophosphate. Basset et al., 99(19) PNAS 12489-94 (2002); Cone & Guroff, 246(4) J. Biol. Chem. 979-85 (1971); Plowman et al., 249(17) J. Biol. Chem. 5559-64 (1974).

Taken together, the data clearly demonstrate that the GCYH-IB proteins catalyze GTP cyclohydrolase I activity, and thus they represent a new structural class of GTP cyclohydrolase enzymes, distinct from the canonical GCYH-I enzyme exemplified by human and *E. coli* FolE. To differentiate these two cyclohydrolase families, the canonical type I cyclohydrolase may be renamed GCYH-IA, that the COG1469 family be named GCYH-IB, and that their corresponding genes be denoted as *folE* and *folE2,* respectively.

### Example7. Over-expression and purification of selenomethionine (SeMet)-labeled N. gonorrhoeae GCYH-IB (Ng-GCYH-IB).

Ng-GCYH-IB was cloned in pET-30 Xa/LIC expression vector (Novagen, San Diego, CA) as described previously. El Yacoubi et al., 2006. SeMet-labeled Ng-GCYH-IB was over-expressed in the *E. coli* methionine auxotroph B834(DE3) (Novagen) following standard methods. Hendrickson et al., 9(5) Eur. Mole. Biol. Org. J. 1665-72 (1990). The His₆-tagged protein was purified on Ni-NTA resin (washed with Tris acetate (100 mM, pH 8.0), KCl (300 mM), β-mercaptoethanol (2 mM), glycerol (10%), and imidazole (20 mM)), and eluted with the same buffer containing 250 mM imidazole. After concentration and dialysis against Tris acetate (50 mM, pH 8.0), KCl (50 mM), and dithiothreitol (4 mM), the His₆ tag was cleaved from the enzyme with Factor Xa as described previously (El Yacoubi et al., 2006) and SeMet-labeled wild-type Ng-GCYH-IB was further purified on a Ni-NTA column and eluted with ten column volumes of Tris-acetate (100 mM, pH 8.0), KCl (300 mM), β-mercaptoethanol (2 mM), Triton X-100 (1%), phenylmethylsulfonyl fluoride (1 mM), and glycerol (10%). After concentrating to 5.5 mg/mL, the protein was dialyzed against Tris-Acetate (50 mM, pH 8.0), KCl (100 mM) and β-mercaptoethanol (5 mM) and further concentrated to 9 mg/mL. Selenium substitution was verified with mass spectrometry using MALDI-TOF analysis.

### Example 8. Crystallization and X-ray data collection.

SeMet-labeled GCYH-IB was crystallized at 20 °C by vapor diffusion in 2 µL sitting drops containing enzyme (9 mg/mL in 50 mM Tris-Acetate, 100 mM KCl, 5 mM BME, pH 8.0), polyethylene glycol 6000 (10-16%), LiCl (1-1.4 M), Tris (50 mM, pH 9.0) and Tris-HCl (50 mM, pH 7.0). The Mn-reconstituted enzyme (10 mg/mL in 50 mM Tris-HCl, 50 mM KCl, 1 mM DTT, pH 8.0) was crystallized under similar conditions but using metal-free reagents (Chelex-100-pretreated Milli-Q water and ultra pure reagents from Hampton, Inc.) with 1-10 mM MnCl₂ and 12 mM sodium azide (as a preservative) added to the protein solution prior to crystallization.

Crystals were cryo-protected in mother liquor plus ethylene glycol (25%) and flash cooled in liquid nitrogen. For the SeMet-labelled enzyme, a three-wavelength selenium dataset was collected at the Advanced Light Source (ALS, Berkeley, CA) beamline 8.2.2. For the GCYH-IB•Mn²⁺ complex, crystals were back-soaked in metal-free reservoir solution for 1 hr prior to cryo protection and a single-wavelength dataset was collected from a single crystal at the Stanford Synchrotron Radiation Laboratory (SSRL, Stanford, CA) beamline 7-1. X-ray data were processed in HKL2000. Otwinowski, Z. & Minor, W. 276 Methods Enzymol., 307-326 (1997).

### Example 9. Structure determination.

The crystal structure of Ng-GCYH-IB was determined by the multi-wavelength anomalous dispersion (MAD) method using selenium as the anomalous scatterer. An initial heavy-atom substructure consisting of 15 selenium sites was identified using the Phenix-hyss program. Adams et al., 11 J. Synchrotron Rad. 53-55 (2004). Heavy atom positions, occupancies, and atomic displacement parameters were then refined in the SHARP maximum likelihood program (Maximum-likelihood heavy atom parameter refinement in the MIR and MAD methods, in METHODS IN ENZYMOLOGY (de La Fortelle & Bricogne, eds., Acad. Press, San Diego, CA, 1997)), and initial phases were calculated using all Se sites. Phases were improved by density modification with DM (Cowtan, 31 Joint CCP4 & ESF-EACBM Newslett. Protein Crystallography, 34-38 (1994)), and SOLOMON (Abrahams & Leslie, 52 Acta Crystallographica 30-42 (1996)), of the CCP4 suite (Collaborative Computational Proj., N.4, D50 Acta Crystallographica 760-63 (1994)), using data in the resolution range 50 Å to 2.2 Å and solvent content of 47%.

With solvent flattening, the mean figure of merit increased from 0.34 to 0.85. The 2.2 Å FoFOM map was calculated and auto-traced in ARP/wARP (Perrakis et al., 6 Nature Structural Bio. 458-63 (1999)), where 418 of the 514 residues in the asymmetric unit were built. The model was manually completed and partially rebuilt in COOT (Emsley & Cowtan, D60 Acta Crystallographica 2126-32 (2004)), utilizing the two-fold non-crystallographic symmetry (NCS) relation. Subsequent crystallographic refinement was carried out with Phenix-refine (Adams et al., 11 J. Synchr. Radiat., 53-55 (2004)), using NCS restraints and Twin Lattice Symmetry parameterization that included 3 domains per monomer. (Isotropic B-group constraints were applied to disordered parts of the model.) Finally, the f and f" were refined in Phenix to final values of -6.4 and 5.6, -5.4 and 4.9, and -5.6 and 3.5, for the peak, inflection, and remote wavelength, respectively. Solvent molecules and ions were added, the structure was further refined in Crystallography and NMR Systems (CNS, Adams et al., 8(5) Curr. Opin. Struct. Biol. 606-11 (1998)), after removal of NCS restraints, and was validated using Procheck software. Vaguine et al., 55 Acta Crystallographica D, 191-205 (1999). The electron density for residues 146-160 and 188-210 of monomer A was weak with refined b-factors >60 Å², indicating that this region is disordered.

The crystal structure of the GCYH-IB•Mn²⁺ complex was determined by direct difference Fourier calculation in which a protein model based on the crystal structure of the Zn²⁺-metallated enzyme was used to calculate phases. The structure was rigid-body refined in CNS, then refined in the program refmac from CCP4 (Michalopoulos, et al. 32 Nucl. Acids Res., 251-54 (2004)), while applying NCS restraints and solvent flattening. NCS restraints were removed in a second refmac run. Ligands and solvent molecules were modeled in Coot (Emsley & Cowtan, 60 Acta Crystallogr. Biol. Crystallogr. D, 2126-32 (2004)), and final refinement in refmac.

**Table 7: GCYH-IB X-ray data collection and phasing statistics**

| **Data collection** | | | |
|---|---|---|---|
| Dataset | Seₚₑₐₖ | Se_{inflection} | Seᵣₑₘₒₜₑ |
| Unit cell (Å) | 91.7, 100.3, 114.1 | 91.7, 100.2, 114.0 | 91.8, 100.4, 114.1 |
| Wavelength (Å) | 0.9793 | 0.9795 | 0.9747 |
| Resolution (Å) | 50.0 -2.20 | 50.0 -2.20 | 50.0 -2.20 |
| Unique reflections (ano) | 26,689 (26,567)¹ | 26,724 (26,675) | 26,490 (25,691) |
| Completeness (%) | 99.8 (99.0) | 99.8 (100.0) | 99.0 (92.0) |
| Redundancy | 5.7 (5.0) | 5.8 (5.7) | 5.4 (3.5) |
| Rmerge (%)² | 8.7 (61) | 7.5 (36.9) | 84 (89.1) |
| <I/σ(I)> | 16.3 | 17.6 | 15.0 |
| ¹ Highest-resolution shell (2.2-2.28 Ǻ) information in parentheses. | | | |
| ² R_{merge}= 100 x (∑*ₕ* ∑ᵢ \| <I(*h*)> - I(*h*)ᵢ \| ) / Σ*ₕ* ∑ᵢ I(*h*)ᵢ, where I(*h*)ᵢ is the i^{th} observation of reflection *h* and <I(*h*)> is the mean intensity of all observations of reflection *h.* | | | |

| **Phasing statistics (29-2.30 Å)** | | | |
|---|---|---|---|
| R_{der}³ | 0.074⁴ | 0.137 | |
| Rₐₙₒₘ⁵ | 0.069 | 0.059 | 0.058 |
| R_{cullis}⁶ (iso⁴ / ano) | 0.49 / 0.73 | 0.83 / 0.72 | 0.84 / 0.86 |
| Phasing power⁷ | | | |
| Centric | 0.06 | 0.11 | 0.16 |
| Acentric iso⁴ | 0.06 | 0.09 | 0.15 |
| Acentric ano | 1.20 | 1.46 | 1.10 |
| FOM⁸, overall | 0.34 | | |
| after DM | 0.83 | | |
| after Solomon | 0.85 | | |
| Number of Se sites | 15 | | |
| ³ R_{der} = ∑*ₕ* \|F_{PH} - F_{P}\| / ∑*ₕ* \|F_{P}\|, where \|F_{P}\| and \|F_{PH}\| are the observed structure factor amplitudes of the native and the derivative, respectively. | | | |
| ⁴ The dispersive differences were treated as isomorphous replacement information where the data collected at wavelength 0.9747 Å are treated as native data (Terwilliger 1994). | | | |
| ⁵ Rₐₙₒₘ = ∑*ₕ* \|F_{PH+} - F_{PH-}\| / ∑*ₕ* \|<F_{PH}>\|, where \|F_{PH+}\| and \|F_{PH-}\| are the Friedel-pair observed structure factor amplitudes of the derivative at a given wavelength, and <F_{pH}> is their average. | | | |
| ⁶ R_{Cullis} = ∑*ₕ* [ \|F_{H}\| - ( \|F_{PH}\| - \|F_{P}\| ) ] / ∑*ₕ* ( \|F_{PH}\| - \|F_{P}\| ), where \|F_{H}\| is the calculated heavy-atom structure factor for reflection *h.* | | | |
| ⁷ PP_{disp} = (1/N_{refl}) ∑*ₕ* [ \|\|F_{pH}\| - F_{P}\|\| / ∫₀^{2π} ( \|F_{PH} - F_{PH}^{calc}\| ) P(ϕ) d(ϕ) ], where P(ϕ) is the probability of a phase value of ϕ for reflection *h.* PPₐₙₒ = (1/N_{refl}) ∑*ₕ* [\|Δ_{obs}^{ANO}\| / ∫₀^{2π} ( \|Δ_{obs}^{ANO} - Δ_{calc}^{ANO}\| ) P(ϕ) d(ϕ), where Δ_{obs}^{ANO} and Δ_{calc}^{ANO} are the Friedel-pair differences in the observed and calculated structure-factor amplitudes, respectively, for reflection *h.* | | | |
| ⁸ FOM: figure of merit. | | | |

| **Structure refinement (50-2.2 Å)⁹** | | | |
|---|---|---|---|
| Number of reflections | | | |
| working / free | 42,833 / 4,631 | | |
| Number of atoms | | | |
| protein / water | 3,827 / 131 | | |
| metal ions, acetate | 2 / 4 | | |
| R-cryst¹⁰ / R-free¹¹ (%) | 0.20 / 0.25 | | |
| Rmsd bond lengths (Å) | 0.007 | | |
| Rmsd bond angles (Å) | 0.825 | | |
| Ramachandran Plot (%) | | | |
| favored | 93.2 | | |
| allowed | 6.8 | | |
| Wilson B-factor (Å²) | 40.3 | | |
| ⁹ During density modification, structure factors were calculated for remote-wavelength data in the resolution range 2.3-2.14 Å. | | | |
| ¹⁰ Crystallographic R-factor = 100 x ( ∑*ₕ* \|\| F_{obs}(*h*) \| - \| F_{calc}(*h*) \|\|) / ∑*ₕ* \| F_{obs}(*h*) \|, where F_{obs}(*h*) and F_{calc}(*h*) are the observed structure factor amplitude and the structure factor amplitude calculated from the model, respectively. | | | |
| ¹¹ The free R-factor was calculated by using 90% of the data. | | | |

**Table 8. GCYH-IB•Mn²⁺ X-ray data collection**

| GCYH-IB•Mn²⁺ | |
|---|---|
| Spacegroup | C222₁ |
| Dataset | |
| Unit cell (Å) | 92.2, 100.4, 113.9 |
| Wavelength (Å) | 0.97607 |
| Resolution (Å) | 30.33 - 2.04 |
| Unique reflections (ano) | 33,675 (3,314)¹ |
| Completeness (%) | 99.8 (99.9) |
| Redundancy | 4.5 (4.5) |
| Rmerge (%) | 7.1 (42.8) |
| <I/σ(I)> | 11.0 |

| | |
|---|---|
| ¹Highest-resolution shell (2.04-2.11 Å) information in parentheses. | |

**Table 9. Structure refinement of GCYH-IB and GCYH-IB•Mn²⁺**

| Structure refinement | | |
|---|---|---|
| | GCYH-IB | GCYH-IB•Mn²⁺ |
| Resolution range | 45.9 - 2.2 Å⁹ | 30.33 - 2.04 |
| Number of reflections | | |
| working / free | 42,833¹⁰ / 4,631 | 31,937 / 1,710 |
| Number of atoms | | |
| protein / water | 3,829 / 133 | 3,829 / 261 |
| active site metal ions | 2 | 2 |
| other ions | 1 | 2 |
| other ligands | 4 | 3 |
| R-cryst¹¹ / R-free¹² (%) | 0.20 / 0.25 | 0.19/0.26 |
| Rmsd bond lengths (Å) | 0.007 | 0.019 |
| Rmsd bond angles (Å) | 0.825 | 1.754 |
| Ramachandran Plot (%) | | |
| favored | 93.2 | 96.9 |
| allowed | 6.8 | 3.1 |
| Wilson B-factor (Å²) | 40.3 | 37.4 |

| | | |
|---|---|---|
| ⁹During density modification, structure factors were calculated for remote-wavelength data in the resolution range 2.3-2.2 Å. ¹⁰Anomalous pairs treated as separate reflections. ¹¹Crystallographic R-factor = 100 x ( ∑h \|\| Fobs(h) \| - \| Fcalc(h) \|\|) / ∑h \| Fobs(h) \|, where Fobs(h) and Fcalc(h) are the observed structure factor amplitude and the structurefactor amplitude calculated from the model, respectively. ¹²The free R-factor was calculated by using 90% and 95% of the data for GCYH-IB and GCYH-IB•Mn²⁺, respectively. | | |

### Example 10. Metal dependencies of GCYH-IB

*Enzyme Activity Assays:* All chemicals were of analytical, metal-free grade from Sigma or Fisher. All solutions were prepared with Milli-Q water (18.2 MΩ) and treated with Chelex-100 (BioRad) to remove contaminating metals. Glassware were soaked in nitric acid (10%), then EDTA (5 mM) and rinsed liberally with Chelex-treated Milli-Q water before use. Activity assays were conducted as described previously (E1 Yacoubi et al, 2006) using either variable concentrations of metal or EDTA (5 mM), and 0.1 mM GTP.

*Apoenzyme Preparation*: Metal ions were removed *from B. subtilis* and *N. gonorrhoeae* GCYH-IB by incubating the purified, recombinant protein (0.5 ml) with EDTA (5 mM, 1 hr, 21 °C) followed by dialysis thrice against a 4-liter solution of Tris-HCl (50 mM, pH 8.0), KC1 (50 mM), EDTA (5 mM) and a few grams of Chelex-100. The resulting apoenzyme was dialyzed thrice against 3 L of Tris-HCl (50 mM, pH 8.0), KC1 (50 mM), and Chelex-100 to remove EDTA and passed through Chelex-100 resin (5 ml protein solution per 3 ml resin) prior to activity assays. Protein concentrations were determined spectrophotometrically.

*Metal Activation Studies*: The *B. subtilis* apoenzyme (2 µM) was incubated with varying concentrations (0.1 µM-4 mM) of metal chlorides (MnCl2, ZnCl2, MgCl2, NiCl2, CaCl2, CdCl2, CoCl2, CuCl2, CoCl3, FeCl3) or Fe(SO4) in standard buffer (100 mM HEPES, pH 8.0, 100 mM KCl) for 10 min at 37 .C, and assayed for activity as described above. All assays involving Fe²⁺ were conducted in an anaerobic chamber with degassed and N2-purged buffers.

*Reconstitution of N*. *gonorrhoeae GCYH-IB with Mn*: The apoenzyme was incubated in the presence of 2-7 molar equivalents of MnCl2 in standard buffer containing 2 mM DTT for 60 min at 25°C. Loosely bound metal was removed either by dialysis twice against Tris-HCl (50 mM, pH 8.0), KCl (50 mM) and DTT (1 mM) for 4 hr at 4°C, filtration on a Sephadex G-25 column, or extensive washing with buffer in an Amicon ultra-centrifugal device. The enzyme was assayed for activity as described above and the metal content analyzed by ICP-MS.

### Example 11. Zinc-dependent regulation of GCYH-IB

*Bioinformatics*: Analysis of the *folE*/*folE2* gene distribution was performed using the SEED annotation environment. Results are made available in the 'Folate biosynthesis' subsystem at http://theseed.uchicago.edu/FIG/index.cgi. Candidate Zur binding sites were identified using the Genome Explorer software (Mironov et al., 2000) by scanning bacterial genomes with two PWMs constructed using the training sets of two different sets of known Zur binding sites in proteobacteria and firmicutes. Panina et al., 100 Proc. Nat'l Acad. Sci. USA, 9912-17 (2003).

*B. subtilis strain construction and growth conditions:* All strains were derived from the *B. subtilis* 168 trpc2attSPβwild-type strain, CU1065, and were grown in LB. Growth curves were done using a Bioscreen CMBR system for 24 hours with OD600 measurements every 10 min. Liquid overnight cultures with antibiotics were used to start pre-cultures that were diluted at mid-log to a normalized OD600 of .01 in a volume of 200 µl in a 100-well honeycomb microtiter plate at which point the growth curve was started. Cultures were incubated at 37°C with shaking at 200 rpm. For selection, antibiotics were added at the following concentrations: erythromycin (1 µg/ml) and lincomycin (25 µg/ml) (for selecting for macrolide-lincosamide-streptogramin B resistance), spectinomycin (100 µg/ml), kanamycin (15 µg/ml).

Mutants in *folE,folE2* and *zur* were constructed using long-flanking-homology polymerase chain reaction (LFH-PCR) as previously described (Butcher & Helmann, 2006). LFH-PCR and chromosomal DNA transformation were used to generate strains expressing various combinations of GCYH-I enzymes. Specifically, strain HB6788 CU1065 *folE*::*mls)* lacks GCYH-IA while its GCYH-IB is repressed, but its gene is still present (designated Δ*folE*). This strain was used for construction of other strains. Strain HB6791 (CU1065 folE::mls zur::kan) expresses only GCYH-IB (designated ΔfolE Δzur). Strain HB6852 (CU1065 *folE*::*mls zur*::*kanfolE2*::*spc)* lacks both GCYH-I isozymes (designated Δ*folE* Δ*zur* Δ*folE2*).

## Claims

1. A crystal of a GCYH-IB•Mn²⁺ polypeptide, wherein the GCYH-IB●Mn²⁺ polypeptide comprises the amino acid sequence of SEQ ID NO: 8, and wherein the GCYH-IB●Mn²⁺ crystal is **characterized by** space group C222₁ and unit cell dimension values: a = 92.2 Å; b = 100.4 Å; and c = 113.9 Å.

## Patentansprüche

1. Kristall eines GCYH-IB•Mn²⁺-Polypeptids, wobei das GCYH-IB•Mn²⁺-Polypeptid die Aminosäuresequenz SEQ ID NO: 8 umfasst und wobei der GCYH-IB•Mn²⁺-Kristall durch die Raumgruppe C222₁ und Dimensionswerte der Elementarzelle: a = 92,2 Å, b = 100,4 Å und c = 113,9 Å, charakterisiert ist.

## Revendications

1. Cristal de polypeptide GCYH-IB•Mn²⁺, dans lequel le polypeptide GCYH-IB•Mn²⁺ comprend la séquence d'acide aminé SEQ ID NO: 8, et dans lequel le cristal de GCYH-IB•Mn²⁺ est **caractérisé par** le groupe spatial C222₁ et les valeurs dimensionnelles cellulaires unitaires suivantes : a = 92,2 Å ; b = 100,4 Å ; et c = 113,9 Å.
